(19)

Europäisches
Patentamt
European
Patent Office
Office européen
des brevets

(11) **EP 1 815 797 A1**

(12) **EUROPEAN PATENT APPLICATION**
published in accordance with Art. 158(3) EPC

(43) Date of publication:
**08.08.2007 Bulletin 2007/32**

(51) Int Cl.:
***A61B 8/12*** *(2006.01)*

(21) Application number: **05809481.4**

(22) Date of filing: **24.11.2005**

(86) International application number:
**PCT/JP2005/021576**

(87) International publication number:
**WO 2006/057296 (01.06.2006 Gazette 2006/22)**

(84) Designated Contracting States:
**DE FR GB**

(30) Priority: **25.11.2004 JP 2004341256**

(71) Applicant: **Olympus Corporation**
**Tokyo 151-0072 (JP)**

(72) Inventors:
• **KAWASHIMA, Tomonao,**
**Olympus Medical Systems Corp**
**Tokyo,**
**151-0072 (JP)**

• **KOMURO Masahiko,**
**Olympus Medical Systems Corp**
**Tokyo,**
**151-0072 (JP)**

(74) Representative: **von Hellfeld, Axel**
**Wuesthoff & Wuesthoff**
**Patent- und Rechtsanwälte**
**Schweigerstrasse 2**
**81541 München (DE)**

(54) **ULTRASONOGRAPHIC DEVICE**

(57)     An ultrasonic diagnostic apparatus includes an ultrasonic observation unit (3) that forms an ultrasonic tomographic image based on an ultrasonic signal obtained by transmission/reception of the ultrasonic wave to/from inside of the living body, a position orientation calculation unit (4) that detects the position and/or the orientation of the ultrasonic tomographic image, a reference image memory (61) that stores the reference image data, a 3D guide image forming circuit (63) that forms a stereoscopic 3D guide image for guiding an anatomical position and/or orientation of the ultrasonic tomographic image based on the reference image data stored in the reference image memory (61) using the position and/or the orientation detected by the position orientation calculation unit (4), and a display unit (9) that displays the 3D guide image formed by the 3D guide image forming circuit (63).

**FIG.20**

9a: DISPLAY SCREEN (3D GUIDE IMAGE AND ULTRASONIC TOMOGRAPHIC IMAGE)
9a1: 3D GUIDE IMAGE
9a2: ULTRASONIC TOMOGRAPHIC IMAGE
73
PANCREAS (LIGHT BLUE)
SUPERIOR MESENTERIC VEIN (PURPLE)
DUODENAL WALL
AORTA
PANCREAS
71: ULTRASONIC TOMOGRAPHIC IMAGE MARKER
DUODENAL WALL (YELLOW)
AORTA (RED)
71a: 12 O'CLOCK DIRECTION MARKER FOR TOMOGRAPHIC IMAGE MARKER

EP 1 815 797 A1

**Description**

Technical Field

The present invention relates to an ultrasonic diagnostic apparatus which forms an ultrasonic tomographic image based on an ultrasonic signal derived from transmission/reception of the ultrasonic wave to/from inside of a living body.

Background Art

Recently an ultrasonic diagnostic apparatus has been increasingly employed to transmit the ultrasonic wave into the living body, and to receive the reflected wave from the living body tissue so as to be converted into an electric signal, based on which the image of the state inside the living body may be observed on the real-time basis.

An operator makes a diagnosis by observing the ultrasonic tomographic image formed by the ultrasonic diagnostic apparatus while estimating the currently observed anatomical position, taking the known anatomical correlations among organs and tissues inside the body into consideration. An ultrasonic diagnostic apparatus configured to display the guide image for indicating the position on the ultrasonic tomographic image observed by the operator has been proposed for the purpose of assisting the aforementioned diagnosis.

The ultrasonic diagnostic apparatus disclosed in Japanese Unexamined Patent Application Publication No. 10-151131 is provided with the image positional relationship display means in which a plurality of images which contain external volume image are input through the image data input unit, and the ultrasonic wave from the probe (ultrasonic probe) outside the body is irradiated so as to obtain the ultrasonic image of a specified diagnostic site. The 2D image (tomographic image) of the position corresponding to the obtained ultrasonic image is further obtained from the image input through the image data input unit such that the tomographic image is laid out or superimposed on the ultrasonic image, or they are alternately displayed at an interval. The use of the aforementioned ultrasonic diagnostic apparatus allows the operator to perform the inspection while comparing the ultrasonic wave image with the tomographic images derived from the X-ray CT scanner or the MRI unit corresponding to the ultrasonic tomographic plane under the inspection.

The ultrasonic diagnostic apparatus disclosed in Japanese Unexamined Patent Application Publication No. 2004-113629 is provided with ultrasonic scan position detection means that detects the position of the site at which the ultrasonic wave is transmitted/received, ultrasonic image forming means that forms an ultrasonic image based on the ultrasonic signal, and control means which derives the anatomical diagram of the site of the subject corresponding to the position detected by the ultrasonic scan position detection means from an image data storage means that contains diagrammatic views of a human body as the guide image so as to be displayed together with the ultrasonic image on the same screen. The ultrasonic diagnostic apparatus as disclosed in Japanese Unexamined Patent Application Publication No. 2004-113629 includes a thin and long flexible ultrasonic probe to be inserted into the body of the subject as means for obtaining the ultrasonic image. The ultrasonic diagnostic apparatus has been proposed, which is provided with the electronic radial scan type ultrasonic endoscope having a group of ultrasonic transducers arranged like an array around the insertion shaft, an electronic convex type ultrasonic endoscope having a group of ultrasonic transducers arranged like a fan at one side of the insertion shaft, and a mechanical scan type ultrasonic endoscope having a piece of the ultrasonic transducer rotating around the insertion shaft as the ultrasonic probes. Each of the aforementioned ultrasonic endoscopes generally includes an illumination window through which the illumination light is irradiated into the body cavity, and an observation window through which the state inside the body cavity is observed, both of which are formed at the tip of the flexible portion inserted into the body cavity.

The ultrasonic diagnostic apparatus disclosed in Japanese Unexamined Patent Application Publication No. 10-151131 is configured to obtain the tomographic image at the position corresponding to the ultrasonic image of the specific diagnostic site. The operator may refer to the obtained tomographic image as the guide image of the ultrasonic image, but is required to estimate the anatomical position not only on the ultrasonic image but also the guide image. Therefore, the guide image is considered as being insufficient to function in guiding the position under observation with the ultrasonic image.

The ultrasonic diagnostic apparatus disclosed in Japanese Unexamined Patent Application Publication No. 2004-113629 is configured to obtain the anatomical graphical image of the site of the subject corresponding to the position detected by the ultrasonic scan position detection means from the image data storage means that contains the graphical data of the human body such that the ultrasonic image is displayed together with the graphical image as the guide image on the same screen. The aforementioned configuration is capable of guiding the position under the observation with the ultrasonic image. However, the disclosure fails to clarify how the anatomical graphical image is created. Accordingly, the aforementioned apparatus is still insufficient to provide the usable guide image.

With the ultrasonic diagnostic apparatus as disclosed in Japanese Unexamined Patent Application Publication No. 2004-113629 using the thin and long flexible ultrasonic probe to be inserted into the subject as the ultrasonic endoscope, the operator is not able to visually confirm the ultrasonic scan plane compared with the ultrasonic diagnostic apparatus as disclosed in Japanese Unexamined Patent Application Publication No. 10-151131 using the ultrasonic probe that irradiates the ultrasonic wave extracorporeally. Accordingly, it is highly required to solve the above-identified problem to display the usable guide image for assisting the diagnosis so as to guide the position under the observation with the ultrasonic image.

In the case where the thin and long flexible ultrasonic probe to be inserted into the subject's body, for example, the ultrasonic endoscope is inserted into the stomach, duodenum and small intestine to observe the organs such as pancreas and gallbladder around duct of pancreas and gallbladder, the organ that exists to the depth of the gastrointestinal rather than being exposed to the portion where the probe is inserted cannot be directly observed through the observation window. When the ultrasonic probe is inserted to observe the aforementioned organs, the anatomical position of the tomographic image is estimated while observing the vascular channel as the index, for example, the aorta, lower great vein, superior mesenteric vessel, superior mesenteric vein, splenic artery, splenic vein and the like. The ultrasonic probe is further operated to change the ultrasonic scan plane for forming the image of the organ around the duct of pancreas and gallbladder on the ultrasonic image while estimating the anatomical position of the tomographic image. Accordingly, the system is especially required to display the vascular channel as the index on the guide image to make the guide image easily identifiable so as to guide the user to the position under observation with the ultrasonic image.

The invention has been made in consideration with the aforementioned circumstances, and it is an object of the present invention to provide an ultrasonic diagnostic apparatus which is capable of displaying the position to be observed with the ultrasonic image using the comprehensible guide image.

Disclosure of Invention

Means for Solving the Problem

For the purpose of realizing the aforementioned object, an ultrasonic diagnostic apparatus according to the first invention includes ultrasonic tomographic image forming means that forms an ultrasonic tomographic image based on an ultrasonic signal obtained by transmission and reception of an ultrasonic wave to and from inside of a living body, detection means that detects a position and/or an orientation of the ultrasonic tomographic image, reference image data storage means that stores reference image data, 3D guide image forming means that forms a stereoscopic 3D guide image for guiding an anatomical position and/or orientation of the ultrasonic tomographic image using the position and/or orientation detected by the detection means based on the reference image data stored in the reference image data storage means, and display means that displays the 3D guide image formed by the 3D guide image forming means.

The ultrasonic diagnostic apparatus of the second invention according to the first invention is provided with extraction means that extracts a specific region from the reference image data stored in the reference image data storage means. The 3D guide image forming means forms the 3D guide image by superimposing an ultrasonic tomographic image marker that indicates a position and an orientation of the ultrasonic tomographic image on the stereoscopic image based on the region extracted by the extraction means.

The ultrasonic diagnostic apparatus of the third invention according to the first invention is further provided with sample point position detection means that detects a position of a sample point of the living body. The 3D guide image forming means forms the 3D guide image by performing a verification between a position of the sample point detected by the sample point position detection means and a position of a characteristic point on the reference image data stored in the reference image data storage means.

In the ultrasonic diagnostic apparatus of the fourth invention according to the third invention, the display means displays at least a portion of the reference image data stored in the reference image data storage means, and further includes characteristic point designation means that designates a position of the characteristic point on the reference image data displayed by the display means.

In the ultrasonic diagnostic apparatus of the fifth invention according to the third invention, the sample point position detection means includes body cavity sample point position detection means that detects a position of the sample point within a body cavity of the living body, and the body cavity sample point position detection means is disposed at a tip portion of an ultrasonic probe inserted into the body cavity.

In the ultrasonic diagnostic apparatus of the sixth invention according to the fifth invention, the detection means serves as the body cavity sample point position detection means.

In the ultrasonic diagnostic apparatus of the seventh invention according to the sixth invention, the sample point position detection means is provided separately from the body cavity sample point position detection means, and further includes body surface sample point position detection means that detects a position of the sample point on a surface of the living body.

In the ultrasonic diagnostic apparatus of the eighth invention according to the third invention, the sample points are set for four points selected from a xiphoid process, a right end of pelvis, a pylorus, a duodenal papilla and a cardia.

The ultrasonic diagnostic apparatus of the ninth invention according to the third invention is further provided with posture detection means that detects a position or a posture of the living body and sample point position correction means that corrects the position of the sample point detected by the sample point position detection means using the position or the posture detected by the posture detection means. The 3D guide image forming means performs a verification between a position of the sample point corrected by the sample point position correction means and a position of the characteristic point on the reference image data stored in the reference image data storage means to form the 3D guide image.

In the ultrasonic diagnostic apparatus of the tenth invention according to the ninth invention, the sample point position detection means includes body cavity sample point position detection means that detects a position of the sample point in the body cavity of the living body, and body surface sample point position detection means that is provided separately from the body cavity sample point position detection means to detect a position of the sample point on a body surface of the living body, wherein the posture detection means serves as the body surface sample point position detection means.

In the ultrasonic diagnostic apparatus of the eleventh invention according to the second invention, the reference image data stored in the reference image data storage means are obtained through an image pickup operation performed by an external image pickup device using a radio-contrast agent, and the extraction means extracts a specific region from the reference image data stored in the reference image data storage means based on a luminance value of the reference image data obtained by a use of the radio-contrast agent.

In the ultrasonic diagnostic apparatus of the twelfth invention according to the second invention, the display means displays at least a portion of the reference image data stored in the reference image data storage means, interest region designation means that designates a portion of the specific region on the reference image data displayed by the display means is provided, and the extraction means extracts the specific region designated by the interest region designation means.

In the ultrasonic diagnostic apparatus of the thirteenth invention according to the first invention, the ultrasonic tomographic image forming means forms an ultrasonic tomographic image based on an ultrasonic signal output from an ultrasonic probe including an insertion portion having a flexibility to be inserted into the body cavity of the living body, and an ultrasonic transducer that is disposed at a tip portion of the insertion portion to transmit and receive the ultrasonic wave to and from inside of the living body.

In the ultrasonic diagnostic apparatus of the fourteenth invention according to the thirteenth invention, the ultrasonic transducer performs a scan operation in a plane orthogonal to an insertion axis of the ultrasonic probe.

In the ultrasonic diagnostic apparatus of the fifteenth invention according to the thirteenth invention, the ultrasonic transducer is formed as an ultrasonic transducer array that electronically performs a scan operation.

In the ultrasonic diagnostic apparatus of the sixteenth invention according to the first invention, the reference image data stored in the reference image data storage means are image data which are classified by respective regions.

In the ultrasonic diagnostic apparatus of the seventeenth invention according to the first invention, communication means that obtains image data picked up by an external image pickup device as the reference image data is further provided, the reference image data storage means stores reference image data obtained by the communication means.

In the ultrasonic diagnostic apparatus of the eighteenth invention according to the seventeenth invention, the communication means is connected to at least one kind of the external image pickup devices via a network through which the reference image data are obtained.

In the ultrasonic diagnostic apparatus of the nineteenth invention according to the seventeenth invention, the external image pickup device is formed as at least one of an X-ray CT scanner, an MRI unit, a PET unit, and an ultrasonic diagnostic unit.

In the ultrasonic diagnostic apparatus of the twentieth invention according to the first to the nineteenth inventions, the display means displays the ultrasonic tomographic image formed by the ultrasonic tomographic image forming means and the 3D guide image formed by the 3D guide image forming means simultaneously.

In the ultrasonic diagnostic apparatus of the twenty-first invention according to the first to the twentieth inventions, the 3D guide image forming means forms the 3D guide image on a real time basis together with formation of the ultrasonic tomographic image performed by the ultrasonic tomographic image forming means based on an ultrasonic signal obtained by transmission and reception of the ultrasonic wave to and from inside of the living body.

The ultrasonic diagnostic apparatus according to the twenty-second invention is provided with ultrasonic tomographic image forming means that forms an ultrasonic tomographic image based on an ultrasonic signal obtained by transmission and reception of an ultrasonic wave to and from inside of a living body, detection means that detects a position and/or an orientation of the ultrasonic tomographic image, reference image data storage means that stores reference image data, a position detection probe including sample point position detection means that detects a position of a sample point of the living body, an ultrasonic endoscope provided with a channel which allows the position detection probe to be inserted therethrough and an optical observation window for obtaining the ultrasonic signal, guide image forming means that forms a guide image to guide an anatomical position and/or orientation of the ultrasonic tomographic image by performing a verification between a position of the sample point detected by the sample point position detection means in a state where the position detection probe protrudes from the channel to be in an optical field range of the optical observation window and a position of a characteristic point on the reference image data stored in the reference image data storage means using a position and/or an orientation detected by the detection means, and display means that displays the guide image formed by the guide image forming means.

According to the first, third and twentieth inventions, the observation point with the ultrasonic image may be displayed using a comprehensible guide image.

As the 3D image is formed through detection not only of the position on the scan plane but also its orientation, the orientation of the radial scan plane changes accompanied with the change in the orientation of the ultrasonic scan plane. This makes it possible to form the 3D guide image further accurately. Therefore, the operator is allowed to accurately observe the interest region with the 3D guide image even if the angle of the scan plane of the ultrasonic endoscope is varied around the interest region while viewing the 3D guide images.

According to the fourth invention, the display means displays at least a portion of reference image data stored in the image data storage means, and the characteristic point designation means is provided for designating the position of the characteristic point on the reference image data displayed by the display means. For example, assuming that the pancreas portion is inspected, the cardia may be set as the characteristic point and a sample point as it is close to the pancreas portion. In the case where the interest region is identified before the operation, the characteristic point and the sample point close to the interest region may be easily set. It is predictable that the calculation of the position and the orientation of the radial scan plane becomes more accurate as the interest region becomes closer to the sample point. The space contained in the convex triangular pyramid defined by the sample points allows further accurate calculation of the position and orientation on the radial scan plane compared with the space outside the triangular pyramid. This makes it possible to form more accurate 3D guide image adjacent to the interest region.

According to the fifth and sixth inventions, the sample point position detection means includes the body cavity sample point position detection means which is disposed at the tip of the ultrasonic endoscope to be inserted into the body cavity and capable of detecting the position of the sample point in the cavity of the body. This allows the user to assume that the sample point in the body cavity follows up the movement of the interest region in the body cavity accompanied with the movement of the ultrasonic endoscope, thus forming more accurate 3D guide image. Moreover, in the case where the pancreas or lung is inspected, the sample point may be obtained around the interest region. It is predictable that the calculation of the position and orientation on the radial scan plane may be more accurate as the interest region is closer to the sample point. It is also predictable that the space contained in the convex triangular pyramid defined by the sample points allows the accurate calculation of the position and orientation on the radial scan plane compared with the space outside the triangular pyramid. Accordingly, the more accurate 3D guide image may be formed around the interest region by obtaining the sample point at the appropriate position in the body cavity.

According to the seventh and tenth inventions, the work for cleaning the ultrasonic endoscope before the operation may be reduced compared with the case where the sample point on the body surface is detected only by the ultrasonic endoscope.

According to the ninth and tenth inventions, the accurate 3D guide image may be formed in spite of the change in the subject's posture while obtaining the sample point or performing the ultrasonic scan.

Incidentally, with the ultrasonic endoscope employed in the ultrasonic diagnostic apparatus, which is formed of the flexible material so as to be inserted into the subject's body cavity, the operator is not allowed to directly view the observation position of the ultrasonic endoscope in the body cavity. Then the affected area is estimated based on the intravital information, and formed into the ultrasonic image so as to be loaded for analysis. The aforementioned operation requires considerably high skill, which has hindered spread of the use of the ultrasonic endoscope in the body cavity. In the thirteenth invention, the ultrasonic image forming means forms the ultrasonic image based on the ultrasonic signal output from the ultrasonic endoscope which includes the flexible portion exhibiting sufficient flexibility to be inserted into the body cavity of the living body, and the ultrasonic transducer which is disposed at the tip of the flexible portion for transmitting and receiving the ultrasonic wave to and from inside of the body. This makes it possible to obtain the 3D guide image which shows the accurate observation site. The ultrasonic diagnostic apparatus for irradiation from inside of the subject's body exhibits medical usability much higher than the ultrasonic diagnostic apparatus for external irradiation. Especially the invention may contribute to the reduction in the inspection time and the learning time of the inexperienced operator.

Employment of the mechanical radial scan for rotating the ultrasonic transducer may cause the flexible shaft to be twisted. The twist of the flexible shaft may further cause angular deviation between the angle output from the rotary encoder and the actual angle of the ultrasonic transducer. This may result in the positional deviation of twelve o'clock direction between the ultrasonic tomographic image and the 3D guide image. In the fifteenth invention, the ultrasonic transducer is configured as the array of the ultrasonic transducer for electronically performing the scan, thus preventing the deviation of twelve o'clock direction.

According to the sixteenth invention, the reference image data stored in the image data storage means are formed of image data classified by the respective areas. Assuming that the data are coded by colors, and preliminarily color-coded reference image data are used to indicate such organs as pancreas, pancreatic duct, choledoch duct, portal so as to be displayed as the 3D guide image, the organs to be indexes on the 3D guide image can be comprehensively observed and the scan plane of the ultrasonic endoscope in the body cavity may be changed while observing the 3D guide image. This may expedite the approach to the interest region such as the lesion, thus contributing to the reduction in the inspection period.

According to the seventeenth, eighteenth and nineteenth inventions, the image data storage means stores the reference image data derived from the external image pickup device, and includes a selector that selects a plurality of kinds of reference image data. As the 3D guide image may be formed of data of the subject, further accurate 3D guide image is expected to be formed. In the present invention, the X-ray 3D helical CT scanner and 3D MRI unit outside the ultrasonic diagnostic apparatus are connected to select a plurality of 2D CT images and 2D MRI images through the network. This makes it possible to select the clearest data of the interest region, resulting in easy observation of the 3D guide image.

According to the twenty-first invention, the 3D guide image forming means forms the ultrasonic image using the ultrasonic signal derived through transmission/reception of the ultrasonic wave to/from inside of the living body together with the 3D guide image in real time. This allows the operator to identify the anatomical site of the living body corresponding to the ultrasonic tomographic image under observation, and further to easily access the intended interest region. Moreover, even if the angle of the scan plane of the ultrasonic endoscope is varied around the interest region, the operator is able to accurately observe the interest region while viewing the 3D guide image.

According to the twenty-second invention, the sample points on the surface of the body cavity may be accurately designated under the visual field of optical image, thus forming accurate guide images.

Brief Description of the Drawings

Fig. 1 is block diagram showing a configuration of an ultrasonic diagnostic apparatus according to embodiment 1 of the present invention.

Fig. 2 is a view showing a configuration of a position detection probe in embodiment 1.

Fig. 3 is a perspective view showing a configuration of a posture detection plate in embodiment 1.

Fig. 4 is a perspective view showing a configuration of a marker stick in embodiment 1.

Fig. 5 is a view schematically showing reference image data stored in the reference image memory in embodiment 1.

Fig. 6 is a view schematically showing the voxel space in embodiment 1.

Fig. 7 is a view showing the orthogonal coordinate axes O-xyz and the orthogonal bases i, j, k which are defined on the transmission antenna in embodiment 1.

Fig. 8 is a flowchart showing a routine for the general operation performed by the ultrasonic image processing unit, mouse, keyboard, and display unit in embodiment 1.

Fig. 9 is a flowchart showing the detail of interest organ extraction process executed in step S 1 shown in Fig. 8.

Fig. 10 is a view showing designation of the interest region on the reference image displayed through loading from the reference image memory in embodiment 1.

Fig. 11 is a view showing the extracted data written in the voxel space in embodiment 1.

Fig. 12 is a flowchart showing the detail of the characteristic point designation process executed in step S2 shown in Fig. 8.

Fig. 13 is a view showing designation of the characteristic point on the reference image displayed through loading from the reference image memory in embodiment 1.

Fig. 14 is a flowchart showing the detail of the sample point designation process executed in step S3 shown in Fig. 8.

Fig. 15 is a view of an optical image on the display screen showing the state in which the position detection probe is to be in contact with the duodenal papilla in embodiment 1.

Fig. 16 is a flowchart showing the detail of the 3D guide image formation and display process executed in step S4 shown in Fig. 8.

Fig. 17 shows a relationship between sample points and the radial scan plane, and a relationship between characteristic points and the ultrasonic tomographic image marker, respectively in embodiment 1.

Fig. 18 is a view showing the ultrasonic tomographic image marker in embodiment 1.

Fig. 19 is a view showing a synthetic data of the ultrasonic tomographic image marker and the extracted data in embodiment 1.

Fig. 20 is a view showing the state in which the ultrasonic tomographic image and the 3D guide image are shown side-by-side on the display screen in embodiment 1.

Fig. 21 is a block diagram showing a configuration of an ultrasonic image processing unit to which an external device is connected in embodiment 2 according to the present invention.

Fig. 22 is a view showing designation of the interest organ shown on the reference image displayed through loading from the reference image memory in embodiment 2.

Fig. 23 is a block diagram showing a configuration of an ultrasonic diagnostic apparatus in embodiment 3 according to the present invention.

Best Mode for Carrying Out the Invention

The embodiments of the present invention will be described referring to the drawings.

(embodiment 1)

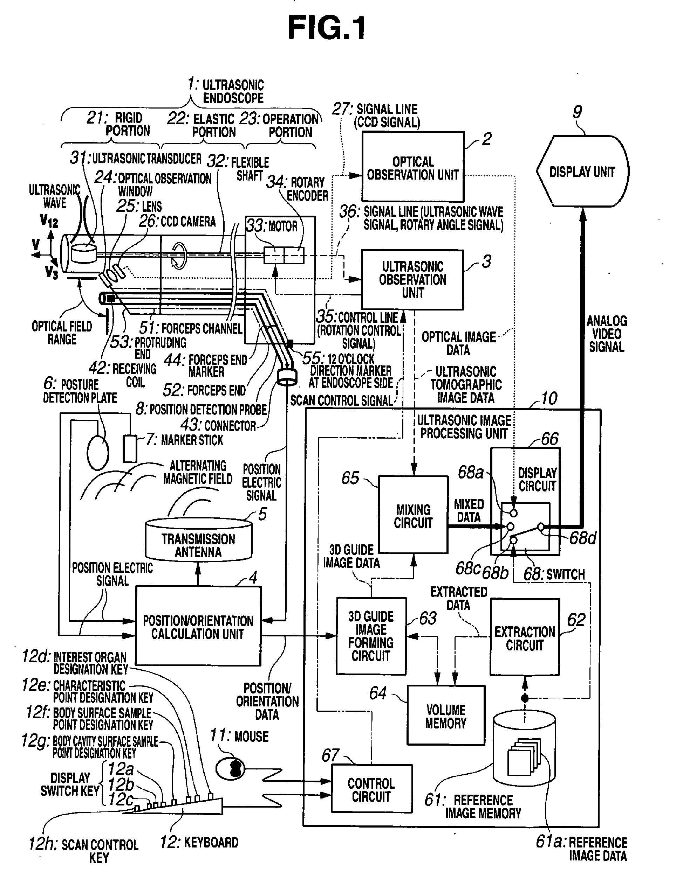

Figs. 1 to 20 show embodiment 1 according to the present invention. Fig. 1 is a block diagram showing a configuration of an ultrasonic diagnostic apparatus.

An ultrasonic diagnostic apparatus of embodiment 1 includes an ultrasonic endoscope 1 as the ultrasonic probe, an optical observation unit 2, an ultrasonic observation unit 3 serving as ultrasonic tomographic image forming means, a position orientation calculation unit 4 serving as detection means, a transmission antenna 5, a posture detection plate 6 serving as body surface sample point position detection means and posture detection means, a marker stick 7 serving as body surface sample point position detection means, a position detection probe 8, a display unit 9 serving as display means, an ultrasonic image processing unit 10, a mouse 11 serving as interest region designation means, and a keyboard 12 serving as interest region designation means, which are electrically coupled with one another via signal lines to be described later.

The ultrasonic endoscope 1 includes a rigid portion 21 provided to the distal-end side and formed of a rigid material, for example, stainless, a long flexible portion 22 connected to the rear end of the rigid portion 21 and formed of a flexible material, and an operation portion 23 provided at the rear end of the flexible portion 22 and formed of a rigid material. The ultrasonic endoscope 1 functions as an insertion portion having the rigid portion 21 and at least a portion of the flexible portion 22 of the aforementioned components inserted into the body cavity.

The rigid portion 21 includes an optical observation window 24 which contains a cover glass, a lens 25 arranged inside the optical observation window 24, a CCD (Charge Coupled Device) camera 26 arranged at the position where an image is formed by the lens 25, and an illumination light emitting window not shown for irradiating the illumination light into the body cavity. The CCD camera 26 is connected to the optical observation unit 2 via a signal line 27.

In the aforementioned configuration, when inside of the body cavity is illuminated with illumination light irradiated from the illumination light emitting window not shown, the image on the surface of the body cavity is formed on the image pickup surface of the CCD camera 26 by the lens 25 through the optical observation window 24. A CCD signal output from the CCD camera 26 is output to the optical observation unit 2 via the signal line 27.

The rigid portion 21 further includes an ultrasonic transducer 31 for transmitting/receiving the ultrasonic wave. The ultrasonic transducer 31 is fixed to one end of a flexible shaft 32 which is provided from the operation portion 23 to the rigid portion 21 via the flexible portion 22. The other end of the flexible shaft 32 is fixed to a rotary shaft of a motor 33 disposed within the operation portion 23.

The rotary shaft of the motor 33 within the operation portion 23 is connected to a rotary encoder 34 that detects a rotation angle of the motor 33 so as to be output.

The motor 33 is connected to the ultrasonic observation unit 3 via a control line 35, and the rotary encoder 34 is connected to the ultrasonic observation unit 3 via a signal line 36, respectively.

In the aforementioned configuration, rotation of the motor 33 causes the ultrasonic transducer 31 to rotate via the flexible shaft 32 in the direction indicated by an outline arrow shown in Fig. 1 around the insertion axis. As the ultrasonic transducer 31 repeatedly transmits/receives the ultrasonic wave while rotating, so-called radial scan is performed. The ultrasonic transducer 31 generates the ultrasonic signal required for forming the ultrasonic tomographic image along the plane perpendicular to the insertion axis of the ultrasonic endoscope 1 (hereinafter referred to as the radial scan plane), and outputs the generated ultrasonic signal to the ultrasonic observation unit 3 via the flexible shaft 32, the motor 33, and the rotary encoder 34, respectively.

The orthogonal bases (unit vector in each direction) V, V3 and V12 fixed to the rigid portion 21 (using characters with normal line thickness instead of using the bold characters hereinafter) are defined as shown in Fig. 1. Specifically, the code V denotes the normal vector on the radial scan plane, V3 denotes the 3 o'clock direction vector on the radial scan plane, and V 12 denotes the 12 o'clock direction vector on the radial scan plane, respectively.

A position orientation calculation unit 4 is connected to a transmission antenna 5, a posture detection plate 6, a marker stick 7, and a long position detection probe 8 via the respective signal lines.

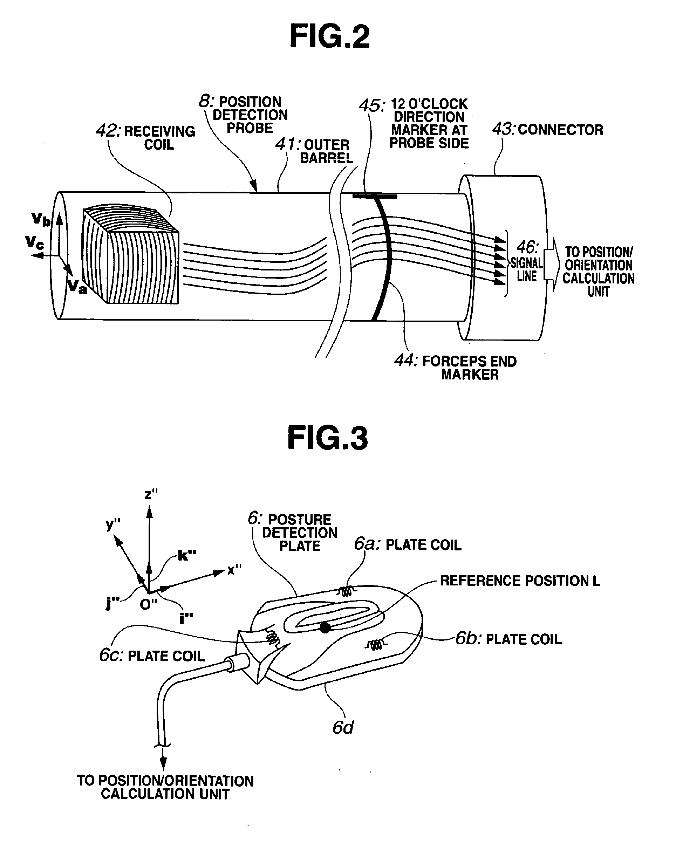

The position detection probe 8 will be described referring to Fig. 2 which shows the configuration thereof.

The position detection probe 8 includes an outer barrel 41 formed of a flexible material. A receiving coil 42 serving as the body cavity sample point position detection means is fixed at the tip side within the outer barrel 41. A connector 43 is disposed at the rear end side of the outer barrel 41. A forceps end marker 44 as the mark along the circumferential direction of the outer barrel 41 for indicating the position of the insertion direction and a 12 o'clock direction marker 45 at the probe side for indicating the position in the circumferential direction are disposed at the rear end on the surface of the outer barrel 41.

The receiving coil 42 is formed by combining three coils each winding axis set to unit vectors Va, Vb and Vc which are fixed to the position detection probe 8 and are orthogonal to one another as shown in Fig. 2. Each of three coils has two poles each of which is connected to one signal line 46 (that is, two signal lines for a single coil). Accordingly, the receiving coil 42 is connected to six signal lines 46 in total. Meanwhile, the connector 43 includes six electrodes (not shown). Each of six signal lines 46 connected to the receiving coil 42 at one end side is connected to the corresponding one of six electrodes at the other end side. Each of six electrodes at the connector 43 is connected to the position orientation calculation unit 4 via the cable (not shown).

The ultrasonic endoscope 1 includes a tubular forceps channel 51 that extends from the operation portion 23 to the rigid portion 21 via the flexible portion 22 as shown in Fig. 1. The forceps channel 51 includes a forceps end 52 as a first opening at the operation portion 23, and a protruding end 53 as a second opening at the rigid portion 21, respectively. The position detection probe 8 is inserted into the forceps channel 51 through the forceps end 52 such that its tip protrudes from the protruding end 53. The protruding end 53 has its opening direction defined such that the tip of the position detection probe 8 protruding from the protruding end 53 is within the range of the optical field range of the optical observation window 24.

The forceps marker 44 is configured such that positions of the tip of the position detection probe 8 and the opening surface of the protruding end 53 coincide with a predetermined positional correlation when the position of the forceps marker 44 coincides with the position of the opening surface of the forceps end 52 in the insertion direction upon insertion of the position detection probe 8 through the forceps end 52 performed by the operator. At this time, the receiving coil 42 is configured to be disposed adjacent to the rotational center of the radial scan performed by the ultrasonic transducer 31. That is, the forceps marker 44 is placed at the position on the surface of the outer barrel 41 such that the receiving coil 42 is placed adjacent to the ultrasonic transducer 31 on the radial scan plane when it coincides with the opening surface of the forceps end 52.

Meanwhile, a 12 o'clock direction marker 55 at the endoscope side is disposed adjacent to the forceps end 52 of the operation portion 23 for the purpose of indicating the position at which the 12 o'clock direction marker 45 at the probe side is coincided. The 12 o'clock direction markers at the probe side and the endoscope side 45 and 55, respectively are configured such that the vector Vc shown in Fig. 2 coincides with the vector V shown in Fig. 1, the vector Va shown in Fig. 2 coincides with the vector V3 shown in Fig. 1, and the vector Vb shown in Fig. 2 coincides with the vector V12 shown in Fig. 1, respectively when the position detection probe 8 is rotated around the vector Vc shown in Fig. 2 while being inserted from the forceps end 52 by the operator until the positions of those markers coincide with each other.

A fixture (not shown) is further provided to the portion around the forceps end 52 of the operation portion 23 for detachably fixing the position detection probe 8 so as not to move in the direction of the insertion axis and so as not to rotate within the forceps channel 51.

The transmission antenna 5 stores a plurality of transmission coils (not shown) each having differently orientated winding axis integrally in a cylindrical enclosure. The plurality of transmission coils stored within the transmission antenna 5 are connected to the position orientation calculation unit 4, respectively.

Fig. 3 is a perspective view showing a configuration of the posture detection plate 6.

The posture detection plate 6 contains three plate coils each formed of a coil having a single winding axis (Fig. 3 is a perspective view showing the plate coils 6a, 6b and 6c, respectively). The orthogonal coordinate axes O"-x"y"z" and orthogonal bases (unit vector in the respective axial direction) i", j" and k" fixed to the posture detection plate 6 are defined as shown in Fig. 3. The plate coils 6a and 6b are fixed within the posture detection plate 6 such that the direction of each winding axis coincides with the direction of the vector i", and the other plate coil 6c is fixed within the posture detection plate 6 such that the direction of the winding axis coincides with the direction of the vector j". The reference position L on the posture detection plate 6 is defined as the gravity center of those three plate coils 6a, 6b and 6c.

The posture detection plate 6 is bound with the subject's body such that the back surface of the posture detection plate 6, which is formed as a body surface contact portion 6d is brought into contact with the surface of the subject's body with an attached belt (not shown).

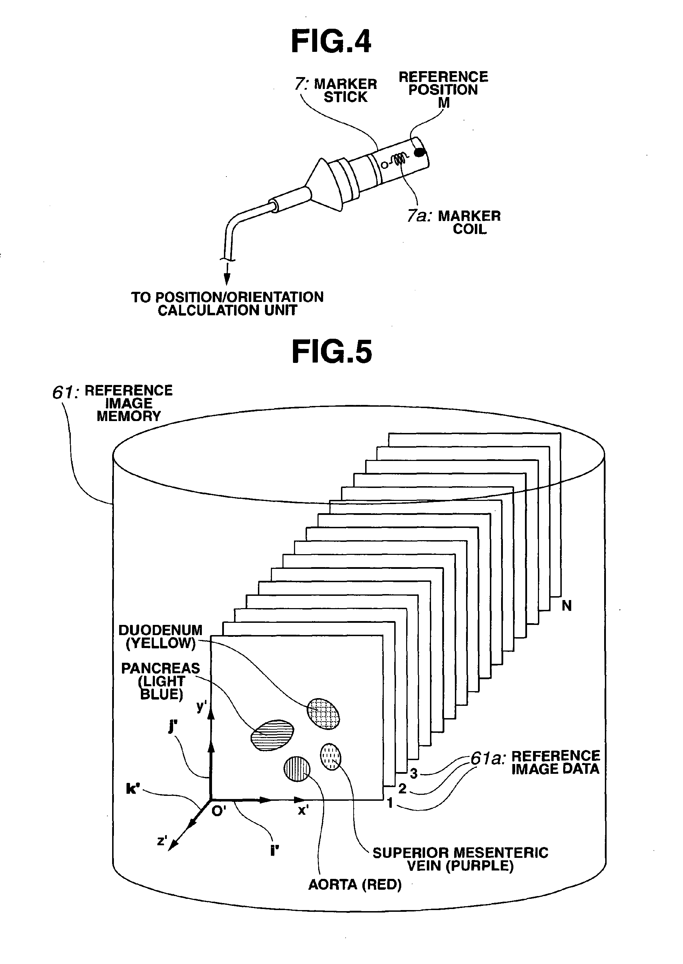

Fig. 4 is a perspective view showing a configuration of the marker stick 7.

The marker stick 7 contains a marker coil 7a formed of a coil with a single winding axis. The marker coil 7a is fixed to the marker stick 7 such that the winding axis coincides with the longitudinal axial direction of the marker stick 7. The tip of the marker stick 7 is defined as the reference position M thereof.

The ultrasonic diagnostic apparatus will be described referring back to Fig. 1.

An ultrasonic image processing unit 10 includes a reference image memory 61 as reference image data storage means, an extraction circuit 62 as extraction means, a 3D guide image forming circuit 63 serving as 3D guide image forming means, sample point position correction means and guide image forming means, a volume memory 64, a mixing circuit 65, a display circuit 66 and a control circuit 67.

The display circuit 66 includes a switch 68 that switches an input. The switch 68 includes three input terminals, that is, 68a, 68b and 68c, and one output terminal 68d. The input terminal 68a is connected to an output terminal (not shown) of the optical observation unit 2. The input terminals 68b and 68c are connected to the reference image memory unit 61 and the mixing circuit 65, respectively. The output terminal 68d is connected to the display unit 9.

The control circuit 67 is connected to the respective components and the respective circuits of the ultrasonic image processing unit 10 via the signal lines (not shown) such that various commands are output. The control circuit 67 is directly connected to the ultrasonic observation unit 3, the mouse 11 and the keyboard 12 outside the ultrasonic image processing unit 10 via the control lines, respectively.

The reference image memory 61 includes a device capable of storing large-volume data, for example, a hard disk drive. The reference image memory 61 stores a plurality of reference image data 61 a as the anatomical image information. Referring to Fig. 5, the reference image data 61 a are obtained by classifying each of square photo data (60 cm x 60 cm) of the frozen body of a human other than the subject sliced in parallel at a pitch of 1 mm with respect to the respective organs by each pixel, which is further color coded to change the attribute. Fig. 5 is a view schematically showing the reference image data 61a stored in the reference image memory 61. Each side of the photo data is set to 60 cm so as to cover substantially the entire transverse section of the body perpendicular to the body axis from the head to leg. The reference image data 61 a within the reference image memory 61 as shown in Fig. 5 are designated with numbers from 1 to N (=integer above 1). The orthogonal coordinate axes O'-x'y'z' and the orthogonal bases (unit vector in the respective axial directions) thereof i', j' and k' fixed to the plurality of reference image data 61a are defined as shown in Fig. 5. That is, an origin O' is defined as the left lower corner of the first reference image data 61a. Based on the origin O', the lateral direction of the image is set to x' axis, the vertical direction of the image is set to y'axis, and the direction of the depth of the images (slices) is set to z' axis. Vectors of the unit length in the respective axial directions are defined as the orthogonal bases i', j' and k', respectively.

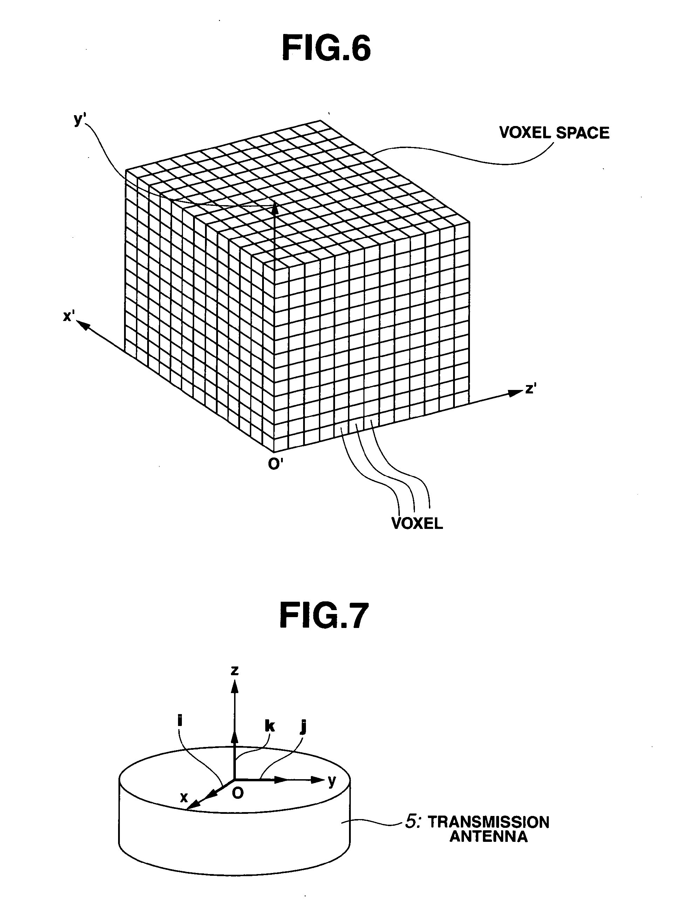

The volume memory 64 is configured to store a large-volume data, and has at least a portion of storage region allocated for the voxel space. The voxel space is formed of memory cells (hereinafter referred to as the voxel) each having addresses corresponding to the orthogonal coordinate axes O'-x'y'z' set for the reference image data 61 a as shown in Fig. 6 schematically showing the voxel space.

The keyboard 12 includes display switch keys 12a, 12b and 12c, an interest organ designation key 12d serving as interest region designation means, a characteristic point designation key 12e, a body surface sample point designation key 12f, a body cavity surface sample point designation key 12g, and a scan control key 12h. When any one of the display switch keys 12a, 12b and 12c is pressed, the control circuit 67 outputs the command to the switch 68 of the display circuit 66 to switch the corresponding input terminal selected from 68a, 68b and 68c. More specifically, the switch 68 is configured to switch to the input terminal 68a when the display switch key 12a is pressed, to switch to the input terminal 68b when the display switch key 12b is pressed, and to switch to the input terminal 68c when the display switch key 12c is pressed, respectively.

The operation of the ultrasonic diagnostic apparatus will be described hereinafter.

Referring to Fig. 1, a dotted line indicates the flow of the signal/data relevant to the optical image (first signal/ data flow), a broken line indicates the flow of the signal/data relevant to the ultrasonic tomographic image (second signal/ data flow), a solid line indicates the flow of the signal/data relevant to the position (third signal/data flow), an alternate long and short dashed line indicates the flow of the signal/data relevant to the reference image data 61a and data formed by processing the reference image data 61a (fourth signal/data flow), a bold solid line indicates the flow of the signal/ data relevant to the final display screen when the 3D guide image data (to be described later) and the ultrasonic tomographic image data (to be described later) are synthesized (fifth signal/data flow), and an alternate long and two short dashed line indicates the flow of the signal/data relevant to the control other those described above (sixth signal/data flow), respectively.

The operation of the ultrasonic diagnostic apparatus of the embodiment will be described referring to the first signal/data flow relevant to the optical image.

The illumination light is irradiated to the optical field range through the light emitting window (not shown) of the rigid portion 21. The CCD camera 26 picks up the image of the object in the optical field range, and outputs the resultant CCD signal to the optical observation unit 2. The optical observation unit 2 creates the image data in the optical field range to be displayed on the display unit 9, and outputs the resultant image data to the input terminal 68a of the switch 68 in the display circuit 66 within the ultrasonic image processing unit 10 as the optical image data.

The operation of the ultrasonic diagnostic apparatus of the present embodiment will be described referring to the second signal/data flow relevant to the ultrasonic tomographic image.

When the operator presses the scan control key 12h, the control circuit 67 outputs the scan control signal for commanding the ON/OFF control for radial scanning to the ultrasonic observation unit 3. Upon reception of the scan control signal from the control circuit 67, the ultrasonic observation unit 3 outputs the rotation control signal for controlling ON/OFF of the rotation to the motor 33. Upon reception of the rotation control signal, the motor 33 rotates the rotary shaft to rotate the ultrasonic transducer 31 via the flexible shaft 32. The ultrasonic transducer 31 1 repeats transmission of the ultrasonic wave and reception of the reflected wave while rotating in the body cavity so as to convert the reflected waves into the electric ultrasonic signals. That is, the ultrasonic transducer 31 performs radial transmission and reception of the ultrasonic wave on the plane perpendicular to the insertion axis of the flexible portion 22 and the rigid portion 21, that is, the radial scanning. The rotary encoder 34 outputs the angle of the rotary shaft of the motor 33 to the ultrasonic observation unit 3 as the rotation angle signal.

The ultrasonic observation unit 3 then drives the ultrasonic transducer 31 and creates a single digitized ultrasonic tomographic image data perpendicular to the insertion axis of the flexible portion 22 with respect to the radial scanning at a single rotation of the ultrasonic transducer 31 based on the ultrasonic signal converted by the ultrasonic transducer 31 from the reflected wave and the rotation angle signal from the rotary encoder 34. The ultrasonic observation unit 3 outputs the created ultrasonic tomographic image data to the mixing circuit 65 of the ultrasonic image processing unit 10. The rotation angle signal from the rotary encoder 34 determines the 12 o'clock direction of the ultrasonic tomographic image data with respect to the ultrasonic endoscope 1 when those data are created. The rotation angle signal thus determines the normal vector V, the 3 o'clock direction vector V3 and 12 o'clock direction vector V12 on the radial scan plane.

The operation of the ultrasonic diagnostic apparatus of the present embodiment will be described referring to the third signal/data flow relevant to the position.

The position orientation calculation unit 4 performs time-shared excitation with respect to the transmission coil (not shown) of the transmission antenna 5 a plurality of times. The transmission antenna 5 forms the alternating magnetic field in the space 7 times in total for three coils that form the receiving coil 42 each having different winding axis, and three plate coils 6a, 6b and 6c of the posture detection plate 6 and the marker coil 7a of the marker stick 7. Meanwhile, the three coils that form the receiving coil 42 each having the different winding axis, three plate coils 6a, 6b and 6c, and the marker coil 7a detect the alternating magnetic field generated by the transmission antenna 5, respectively such that the detected magnetic field is converted into the position electric signal to be output to the position orientation calculation unit 4.

The position orientation calculation unit 4 calculates the positions and the direction of the winding axis of three coils whose winding axes are orthogonal to one another of the receiving coil 42 based on the respective position electric signals time-shared input, and further calculates the position and orientation of the receiving coil 42 using the calculated values. The detailed explanation with respect to the calculated values relevant to the position and orientation of the receiving coil 42 will be described later.

The position orientation calculation unit 4 calculates positions of the three plate coils 6a, 6b and 6c of the posture detection plate 6 and the direction of the winding axis based on the respective time-shared input position electric signals. The position orientation calculation unit 4 calculates the gravity center of the three plate coils 6a, 6b and 6c, that is, the reference position L of the posture detection plate 6 using the calculated values of positions of the three plate coils 6a, 6b and 6c. The position orientation calculation unit 4 calculates the orientation of the posture detection plate 6 using the calculated values of the direction of the winding axes of three plate coils 6a, 6b and 6c. The detailed explanation of the calculated values relevant to the reference position L and orientation of the posture detection plate 6 will be described later.

The position orientation calculation unit 4 calculates the position of the marker coil 7a of the marker stick 7 and the direction of the winding axis. The distance between the marker coil 7a and the tip of the marker stick 7 is preliminarily set to a designed value that is stored in the position orientation calculation unit 4. The position orientation calculation unit 4 calculates the reference position M of the marker coil 7a based on the calculated position of the marker coil 7a, the direction of the winding axis, and the distance between the marker coil 7a and the tip of the marker stick 7 as the predetermined designed value. The detailed explanation with respect to the reference position M of the marker coil 7a will be described later.

The position orientation calculation unit 4 outputs the thus calculated position and orientation of the receiving coil 42, the reference position L and orientation of the posture detection plate 6, and the reference position M of the marker coil 7a to the 3D guide image forming circuit 63 of the ultrasonic image processing unit 10 as the position/orientation data.

In the embodiment, the origin O is defined to be on the transmission antenna 5, and the orthogonal coordinate axes O-xyz, and the orthogonal bases (unit vector in the respective axial direction) thereof i, j and k are defined on the actual space where the subject is inspected by the operator as shown in Fig. 7. Fig. 7 is a view showing the orthogonal coordinate axes O-xyz and the orthogonal bases i, j and k defined on the transmission antenna 5.

Then, the contents of the position/orientation data are provided as the function of time t as following components (1) to (6).

(1) direction component at a position C(t) of the receiving coil 42 (position of the receiving coil 42 is set to C) on the orthogonal coordinate axes O-xyz of the position vector OC(t);
(2) direction component on the orthogonal coordinate axes O-xyz of the direction unit vector Vc(t) indicating the first winding axis direction of the receiving coil 42;
(3) direction component on the orthogonal coordinate axes O-xyz of the direction unit vector Vb(t) indicating the second winding axis direction of the receiving coil 42;
(4) direction component on the orthogonal coordinate axes O-xyz of the position vector OL(t) at the reference position L(t) of the posture detection plate 6;
(5) a 3 x 3 rotating matrix T(t) indicating the orientation of the posture detection plate 6; and
(6) direction component on the orthogonal coordinate axes O-xyz of the position vector OM(t) at the reference position M(t) of the marker stick 7.

As the receiving coil 42, the posture detection plate 6 and the marker stick 7 move within the space, and the positions and orientations of those elements change with time t accordingly, the position/orientation data are defined as the function of time t. The position orientation calculation unit 4 normalizes each length of Vc(t) and Vb(t) preliminarily to a unit length so as to be output.
The rotating matrix T(t) within the aforementioned position/orientation data is formed as the matrix that represents the orientation of the posture detection plate 6 with respect to the orthogonal coordinate axes O-xyz shown in Fig. 7. Strictly, the (m,n) component tmn(t) of the rotating matrix T(t) is defined by the following formula 1:

[Formula 1]

$$t_{mn}(t) = e''_m \cdot e_n$$

where the code "·" in the right side denotes the inner product.
The code "en" in the right side of the formula 1 denotes any one of the base vectors i, j and k of the orthogonal coordinate axes O-xyz, which is defined by the following formula 2.

[Formula 2]

$$e_n = \begin{cases} \mathbf{i} & (n=1) \\ \mathbf{j} & (n=2) \\ \mathbf{k} & (n=3) \end{cases}$$

Moreover, the code "e"m" in the right side of the formula 1 denotes any one of the base vectors (orthogonal bases) i", j" and k" of the orthogonal coordinate axes O"-x"y"z" fixed to the posture detection plate 6 as shown in Fig. 3, which is defined by the following formula 3.

[Formula 3]

$$e''_m = \begin{cases} \mathbf{i''} & (m=1) \\ \mathbf{j''} & (m=2) \\ \mathbf{k''} & (m=3) \end{cases}$$

As described above, the posture detection plate 6 is supposed to be bound to the subject's body with the belt. This means that the orthogonal coordinate axes O"-x"y"z" are fixed to the body surface of the subject. The position of the origin O" may be arbitrarily set so long as the positional relationship with the posture detection plate 6 is fixed. In the present embodiment, it is set to the reference position L(t) of the posture detection plate 6. For easy understanding, the orthogonal coordinate axes O"-x"y"z" and the orthogonal bases i", j" and k" thereof are positioned apart from the posture detection plate 6 as shown in Fig. 3. It is clearly understood that the time dependency of the rotating matrix T(t) is attributable to the time dependency of the base vectors (orthogonal bases) i", j" and k".

The following formula 4 is established by the definition of T(t).

[Formula 4]

$$(\mathbf{i} \quad \mathbf{j} \quad \mathbf{k}) = (\mathbf{i}'' \quad \mathbf{j}'' \quad \mathbf{k}'')\mathbf{T}(t)$$

Moreover, the rotating matrix T(t) is formed on the assumption that the orthogonal coordinate axes O"-x"y"z" virtually fixed on the posture detection plate 6 coincide with the orthogonal coordinate axes O-xyz which is subjected to the rotations at the angle ψ around the z axis, at the angle ϕ around the y axis, and at the angle θ around the x axis in the aforementioned order using so-called Euler angles θ, ϕ, ψ. The rotating matrix T(t) may be expressed by the following formula 5.

[Formula 5]

$$\mathbf{T}(t) = \begin{pmatrix} \cos\phi\cos\psi & \cos\phi\sin\psi & -\sin\phi \\ \sin\theta\sin\phi\cos\psi - \cos\theta\sin\psi & \sin\theta\sin\phi\sin\psi + \cos\theta\cos\psi & \sin\theta\cos\phi \\ \cos\theta\sin\phi\cos\psi + \sin\theta\sin\psi & \cos\theta\sin\phi\sin\psi - \sin\theta\cos\psi & \cos\theta\cos\phi \end{pmatrix}$$

Here, each of those angles θ, ϕ, ψ is the function of the time t (θ(t), ϕ(t), ψ(t)) as the posture of the subject changes as passage of time. The rotating matrix T(t) is the orthogonal matrix, and the transposed matrix thereof is equivalent to the inverse matrix.

The fourth flow of the reference image data 61 a and the data formed by processing the reference image data 61 a will be described later together with the detailed description with respect to the operation of the ultrasonic image processing unit 10.

The operation of the ultrasonic diagnostic apparatus of the present embodiment will be described referring to the fifth signal/data flow relevant to the fianl display screen where the ultrasonic tomographic image data and the 3D guide image data (described later) are synthesized.

The mixing circuit 65 creates mixed data to to be displayed by arranging the ultrasonic tomographic image data from the ultrasonic observation unti 3 and the 3D guide image data from the 3D guide image forming circuit 63 (described later).

The display circuit 66 converts the mixed data into the analog video signal.

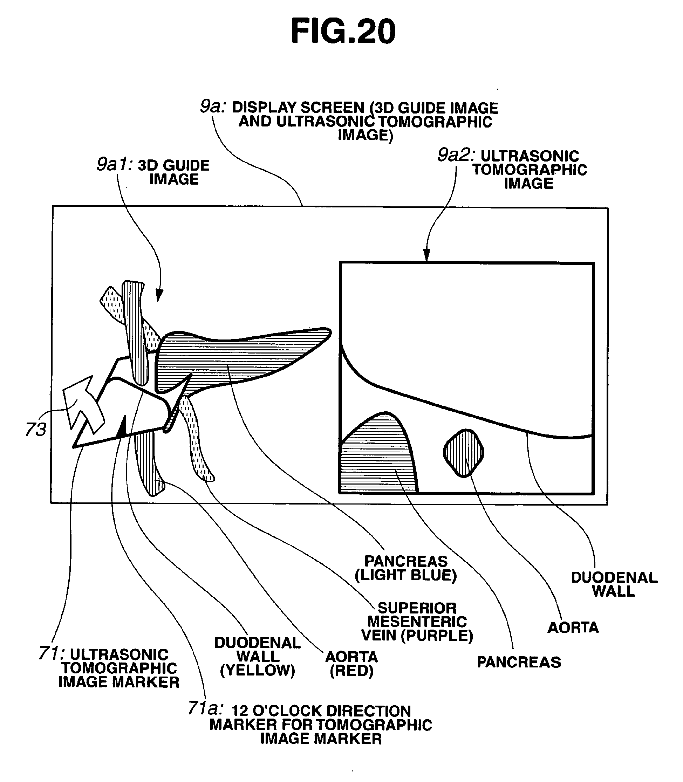

Based on the analog video signal, the display unit 9 arranges the ultrasonic tomographic image and the 3D guide image so as to be displayed side-by-side (the example is shown in Fig. 20).

The operation of the ultrasonic diagnostic apparatus of the embodiment will be described referring to the sixth signal/data flow relevant to the control.

The 3D guide image forming circuit 63, the mixing circuit 65, the reference image memory 61, and the display circuit 66 in the ultrasonic image processing unit 10 are controlled in response to the command from the control circuit 67. The detailed explanation with respect to the control will be described together with the explanation of the operation of the ultrasonic image processing unit 10.

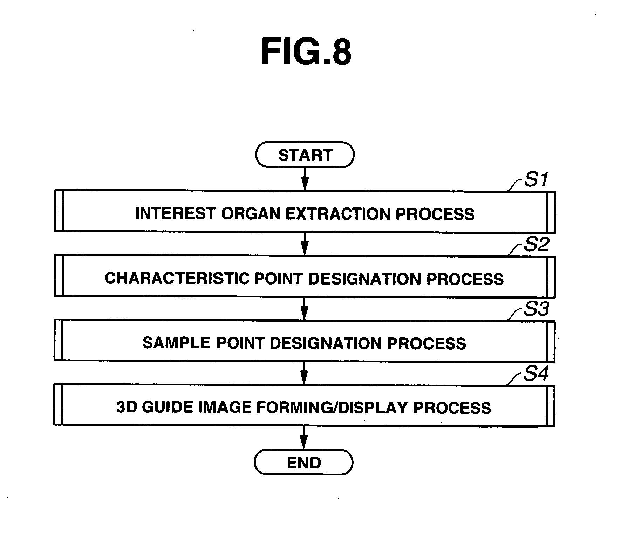

Fig. 8 is a flowchart showing the routine of the general operations performed by the ultrasonic image processing unit 10, the mouse 11, the keyboard 12 and the display unit 9.

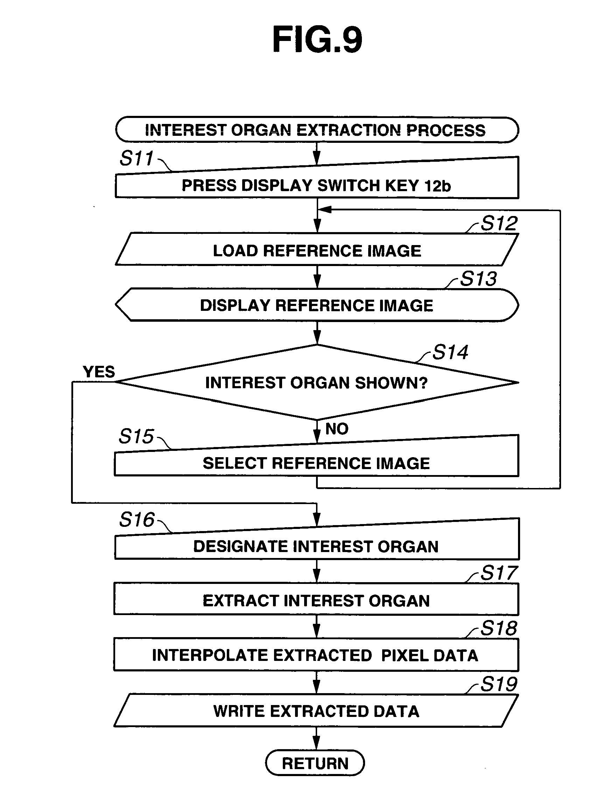

Upon start of the routine, the interest organ extraction process is performed (step S1). Then the characteristic point designation process (step S2), the sample point designation process (step S3) and the 3D guide image formation/ display process (step S4) are performed, respectively, and then the routine ends. The detailed explanations of those steps from S 1 to S4 will be described later referring to Figs. 9, 12, 14 and 16.

Fig. 9 is a flowchart showing the detail of the interest organ extraction process performed in step S 1 shown in Fig. 8.

In the present embodiment, the explanation will be made with respect to extraction of the interest organ, for example, pancreas, aorta, superior mesenteric vein and duodenum.

Upon start of the routine, when the control circuit 67 detects that the operator has pressed the display switch key 12b on the keyboard 12, the switch 68 of the display circuit 66 is switched to the input terminal 68b (step S 11).

Next, the control circuit 67 allows the display circuit 66 to load the reference image data 61a from the reference image memory 61 (step S12). At this time, the control circuit 67 controls to load the first reference image data 61a.

Subsequently, the display circuit 66 converts the first reference image data 61a into the analog video signal, and the converted reference image is output to the display unit 9. Accordingly, the display unit 9 displays the reference image (step S 13).

Thereafter, it is confirmed by the operator whether the interest organ is shown in the reference image on the display screen of the display unit 9 (step S 14).

If the interest organ is not shown, the operator presses a predetermined key on the keyboard 12, or clicks the menu on the screen with the mouse 11 such that the reference image data 61a to be displayed becomes the other reference image data 61a (step S15). Here, specifically, the operator commands to select the reference image data 61a designated with the subsequent number.

Thereafter, the control circuit 67 returns to step S 12 where the aforementioned process is repeatedly performed.

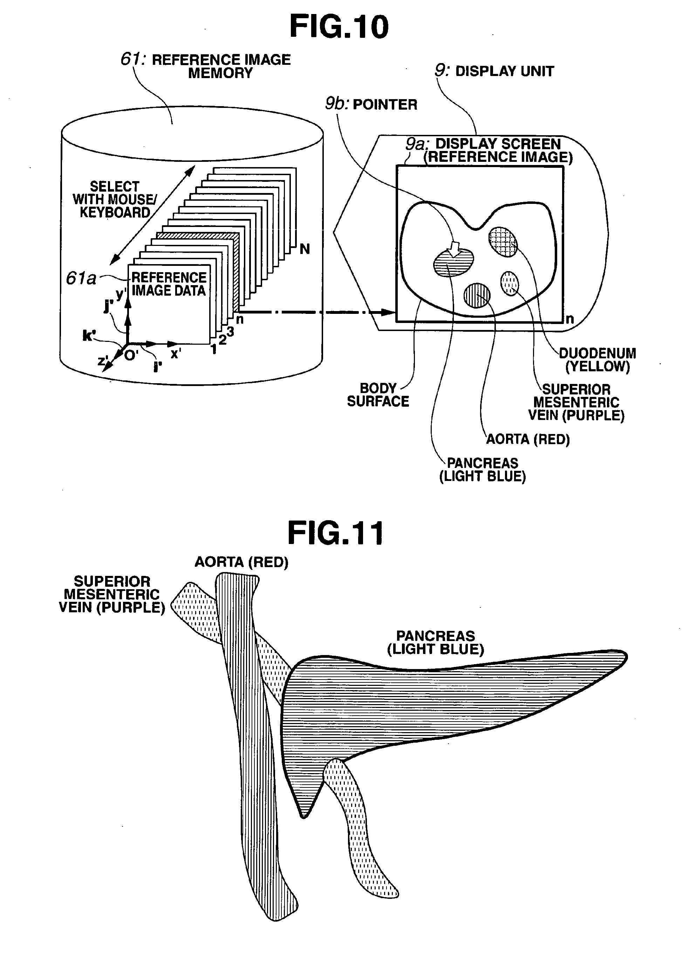

Meanwhile, if the interest organ is shown in step S 14, the operator designates the interest organ on the display screen of the display unit 9 (step S16). The aforementioned state will be described referring to Fig. 10. Fig. 10 is a view in which the interest organ shown in the reference image loaded from the reference image memory 61 to be displayed is designated.

Fig. 10 shows the reference image corresponding to the nth (n = integer ranging from 1 to N) reference image data 61a is displayed on the display screen 9a of the display unit 9. On the display screen 9a, the reference image with the size that covers substantially entire transverse section of the human body perpendicular to the body axis is color-coded by the respective organs at every pixel. In the present embodiment shown in Fig. 10, the pancreas, aorta, superior mesenteric vein and duodenum are displayed in light blue, red, purple and yellow, respectively. A pointer 9b that can be moved on the screen with the mouse 11 is displayed on the display screen 9a. The operator moves the pointer 9b to such interest organs as the pancreas, aorta, superior mesenteric vein and duodenum sequentially, and presses the interest organ designation key 12d on the keyboard 12 on those displayed interest organs so as to be designated.

The pixel corresponding to the designated interest organ is extracted from all the reference image data 61a, that is, from the first to the Nth reference image data 61a by the extraction circuit 62 (step S 17). For example, in the case where the pancreas, aorta, superior mesenteric vein and duodenum are designated as the interest organs in step S16, pixels of such colors as light blue, red, purple and yellow are extracted from all the reference image data 61a.

Thereafter, the extraction circuit 62 interpolates the extracted data at each of the reference image data 61a so as to allocate the data to all the voxels in the voxel space (step S 18). The data extracted in step S 17 and the pixel data interpolated in step S 18 will be referred to as extracted data.

Then, the extraction circuit 62 writes the extracted data into the voxel space within the volume memory 64 (step S 19). At this time, the extraction circuit 62 writes the extracted data to the voxel at the address corresponding to the coordinates on the orthogonal coordinate axes O'-x'y'z' at each pixel. The extraction circuit 62 allocates the colored pixel data for the voxel corresponding to the pixel extracted in step S 17, the data obtained by interpolating the pixel for the voxel between pixels extracted in step S 17, and zero (transparent) for the rest of the voxels. Thus, the extraction circuit 62 allocates the data for all the voxels in the volume space to form the dense data.

Fig. 11 is a view showing the extracted data written in the voxel space. In Fig. 11 as the view corresponding to the case where the pancreas, aorta, superior mesenteric vein, and duodenum are designated as the interest organs, the duodenum is omitted for the purpose of clarifying shapes of the respective interest organs.

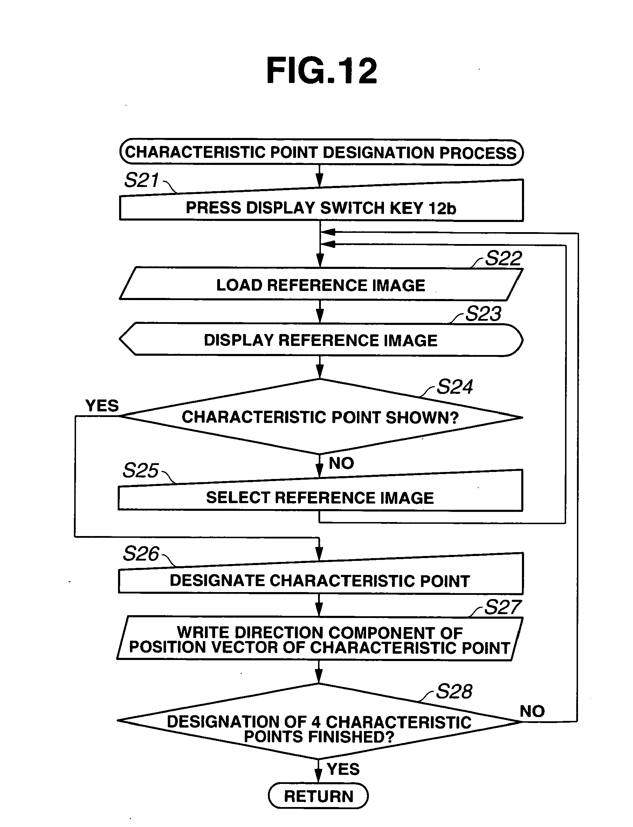

Fig. 12 is a flowchart showing the detail of the characteristic point designation process executed in step S2 shown in Fig. 8.

In the present embodiment, the process for designating the xiphoid process, right end of pelvis, pylorus and duodenal papilla as the characteristic points will be described.

Firstly steps S21 to S23 which are the same as steps S 11 to S 13 shown in Fig. 9 are executed.

Then the operator confirms whether the characteristic points are shown in the reference image displayed on the display screen 9a of the display unit 9 (step S24).

If the characteristic points are not shown, the process in step S25 which is the same as step S 15 shown in Fig. 9 is executed.

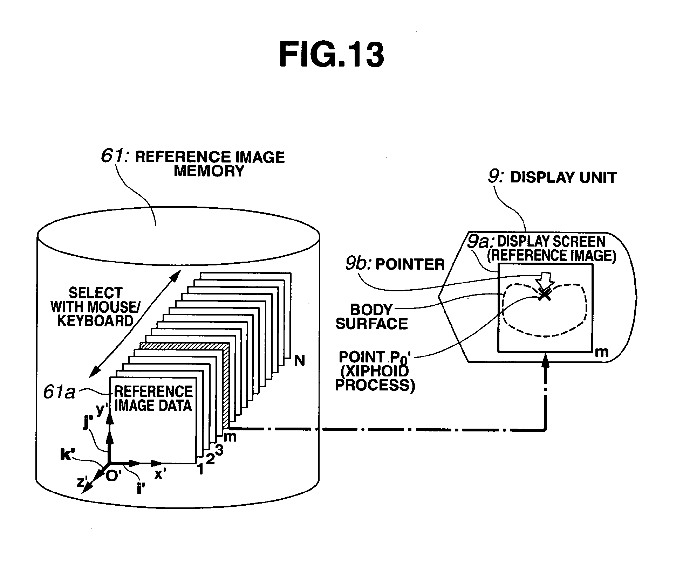

If the characteristic points are shown in step S24, the operator designates the characteristic points shown on the display screen 9a of the display unit 9 (step S26). The aforementioned designation will be described referring to Fig. 13. Fig. 13 is the view showing designation of the characteristic points on the displayed reference image loaded from the reference image memory 61.

Fig. 13 shows the reference image corresponding to the mth (m = integer ranging from 1 to N) reference image data 61a displayed on the display screen 9a of the display unit 9. On the display screen 9a, the reference image with the size that covers substantially entire transverse section of the human body perpendicular to the body axis is color-coded by the respective organs at each pixel. The example in Fig. 13 shows the xiphoid process at a point P0' (the first position of the characteristic point is defined as P0', and the subsequent positions will be defined as P1', P2', P3' and the like). The display screen 9a displays the pointer 9b moved on the screen by the mouse 11. The operator moves the pointer 9b to the interest characteristic point and presses the characteristic point designation key 12e on the keyboard thereon to designate the characteristic point.

The extraction circuit 62 writes the direction component on the orthogonal coordinate axes O'-x'y'z' of the position vector of the designated characteristic point in the volume memory 64 (step S27).

Thereafter, the control circuit 67 determines whether designation of four characteristic points has been finished (step S28). If the designation has not been finished, the process returns to step S22 where the aforementioned process is repeatedly executed.

Meanwhile, if it is determined that the designation of the four characteristic points has been finished in step S28, the process returns from the characteristic point designation process to the process as shown in Fig. 8.

The characteristic points designated by the operator will be designated as P0' P 1', P2' and P3' in the designation order, respectively. In the present embodiment, the xiphoid process, right end of pelvis, pylorus and duodenal papilla will be designated as P0', P1', P2' and P3', respectively.

The extraction circuit 62 writes the respective direction components xP0', yP0' and zP0' of the position vector O'P0' on the orthogonal coordinate axes O'-x'y'z', the respective direction components xP1', yP1' and zP1' of the position vector O'P1' on the orthogonal coordinate axes O'-x'y'z', the respective direction components xP2', yP2' and zP2' of the position vector O'P2' on the orthogonal coordinate axes O'-x'y'z', and the respective direction components xP3', yP3' and zP3' on the orthogonal coordinate axes O'-x'y'z' of the position vector O'P3' in the volume memory 64 at every designation of the characteristic points, respectively by each characteristic point. As described above, each side of each of the reference image data 61 a is set to a constant value of 60 cm. Those images are aligned in parallel at the constant pitch of 1 mm. The extraction circuit 62 is allowed to calculate the respective direction components.

The following formulae 6 to 9 are established as the respective direction components on the orthogonal coordinate axes O'-x'y'z' may be defined as described above.

[Formula 6]

$$\mathbf{O'P_0'} = x_{P0'}\mathbf{i'} + y_{P0'}\mathbf{j'} + z_{P0'}\mathbf{k'}$$

[Formula 7]

$$\mathbf{O'P_1'} = x_{P1'}\mathbf{i'} + y_{P1'}\mathbf{j'} + z_{P1'}\mathbf{k'}$$

[Formula 8]

$$\mathbf{O'P_2'} = x_{P2'}\mathbf{i'} + y_{P2'}\mathbf{j'} + z_{P2'}\mathbf{k'}$$

[Formula 9]

$$\mathbf{O'P_3'} = x_{P3'}\mathbf{i'} + y_{P3'}\mathbf{j'} + z_{P3'}\mathbf{k'}$$

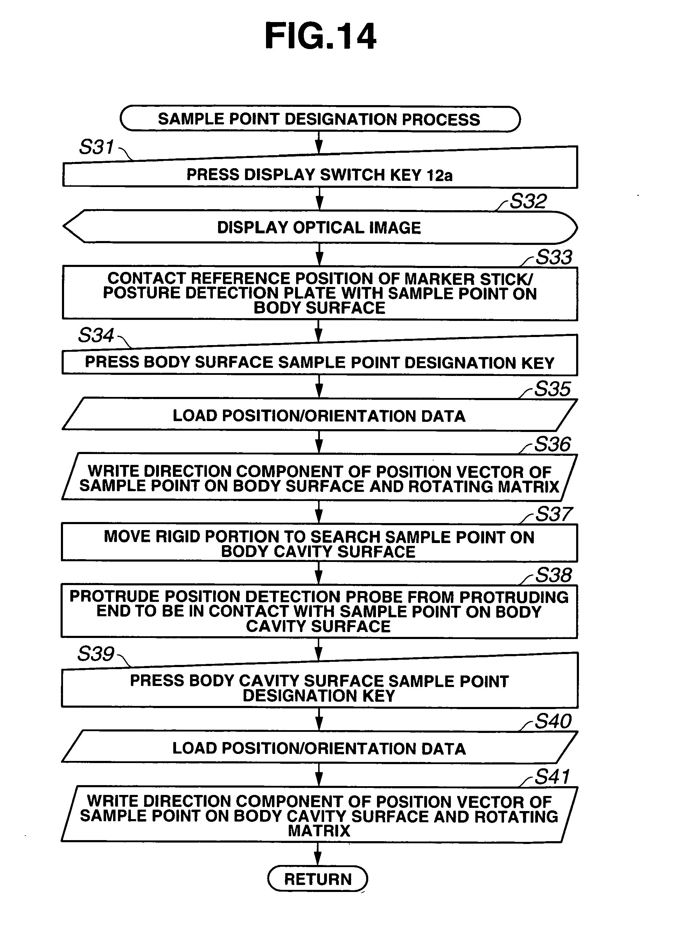

Fig. 14 is a flowchart of the sample point designation process executed in step S3 shown in Fig. 8.

The "sample points" P0, P1, P2 and P3 are points on the body surface or the body cavity surface of the subject anatomically corresponding to the "characteirstic points" P0', P1', P2' and P3', respectively. In the present embodiment, likewise the characteristic points, designation of the xiphoid process, right end of pelvis, pylorus and duodenal papilla as the sample points will be described hereinafter.

As described above, the pairs of the characteirstic point and the sample point of P0' and P0, P1' and P1, P2' and P2, and P3' and P3 indicate the xiphoid process, right end of pelvis, pylorus and duodenal papilla, respectively. In this case, the sample points P0 and P1 are on the body surface of the subject, and the sample points P2 and P3 are on the body cavity surface of the subject.

Upon start of the routine, the control circuit 67 detects that the operator has pressed the display switch key 12a on the keyboard 12, and switches the switch 68 of the display circuit 66 to the input temrinal 68a (step S31).

Next, the display circuit 66 converts the optical image data from the optical observation unit 2 into the analog video signal, and outputs the converted optical image data to the display unit 9. The resultant optical image is displayed on the display unit 9 (stesp S32).

Then the subject is made lying on the left side by the operator, that is, in the left lateral decubitus position. The operator puts the posture detection plate 6 on the subject using the attached belt such that the reference position L of the posture detection plate 6 is overlapped with the position of the xiphoid process of the subject's costa. The operator further brings the reference position M at the tip of the marker stick 7 into contact with the right end of pelvis of the subject (step S33).

The operator presses the body surface sample point designation key 12f (step S34). The time when the above operation is performed is defined as t1.

Thereafter, the 3D guide image forming circuit 63 loads the position/orientation data from the position orientation calculation unit 4 (step S35).

The 3D guide image forming cirucit 63 obtains the respective direction components of the position vector OL (t1) at the reference position L (t1) of the posture detection plate 6 on the orthogonal coordinate axes O-xyz, and the respective direction components of the position vector OM(t1) at the reference position M (t1) of the marker stick 7 on the orthogonal coordinate axes O-xyz.

As the position vector OP0(t1) of the position P0 of the xiphoid process (whose direction components are xP0 (tl), yP0(t1), zP0(t1) on the orthogonal coordinate axes O-xyz) is the same as the OL(t1), the following formula 10 may be expressed.

[Formula 10]

$$\mathbf{OP_0}(t1) = x_{P0}(t1)\mathbf{i} + y_{P0}(t1)\mathbf{j} + z_{P0}(t1)\mathbf{k} = \mathbf{OL}(t1)$$

As the position vector OP1(t1) of the position P1 of the right end of pelvis (whose direction components are xP1(t1), yP1(t1), zP1(t1) on the orthogonal coordinate axes O-xyz) is the same as the OM(t1), the following formula 11 may be expressed.

[Formula 11]

$$\mathbf{OP_1}(t1) = x_{P1}(t1)\mathbf{i} + y_{P1}(t1)\mathbf{j} + z_{P1}(t1)\mathbf{k} = \mathbf{OM}(t1)$$

Moreover, the 3D guide image forming circuit 63 simultaneously obtains the rotating matrix T(t1) that indicates the orientation of the posture detection plate 6 from the position orientation calculation unit 4. The rotating matrix T is used for correcting the change in each position of the respective sample points P0, P1, P2 and P3 caused by the change in the posture of the subject. The process of correcting the sample point will be described later.

The 3D guide image forming cirucit 63 thus has been able to obtain the respective direction components of the OP0(t1) and OP1(tl) on the orthogonal coordinate axes O-xyz at the time t1, and the rotating matrix T(t1).

Next, the 3D guide image forming circuit 63 writes the respective direction components of the OPO(t1) and OP1(t1) at the time t1 on the orthogonal coordinate axes O-xyz, and the rotating matrix T(t1) in the volume memory 64 (step S36).

The operator then inserts the rigid portion 21 and the flexible portion 22 into the body cavity of the subject, and searches the sample point while observing the optical image to move the rigid portion 21 adjacent to the sample point (pylorus) (step S37).

Then the operator inserts the position detection probe 8 from the forceps end 52 while observing the optical image such that the tip protrudes from the protruding end 53. The operator brings the tip of the position detection probe 8 into contact with the sample point (pylorus) under the optical visual field (step 38).

When the tip of the position detection probe 8 contacts with the sample point (pylorus), the operator presses the body cavity surface sample point designation key 12g on the keyboard 12 (step S39). The time when the aforementioned operation is performed is defined as t2.

Then, the 3D guide image forming circuit 63 loads the position/orientation data from the position orientation calculation unit 4 (step S40).

The 3D guide image forming cirucit 63 obtains the respective direction components of the position vector OC (t2) at the position C(t2) of the receiving coil 42 at the tip of the position detection probe 8 on the orthogonal coordinate axes O-xyz from the position/orientation data.

As the position vector OP2(t2) of the position P2 of the pylorus (whose direction components are xP2(t2), yP2 (t2), zP2(t2) on the orthogonal coordinate axes O-xyz) is the same as the OC(t2), the following formula 12 may be expressed.

[Formula 12]

$$OP_2(t2) = x_{P2}(t2)\mathbf{i} + y_{P2}(t2)\mathbf{j} + z_{P2}(t2)\mathbf{k} = OC(t2)$$

At this time, the 3D guide image forming circuit 63 simultaneously obtains the respective direction components of the position vector OL(t2) at the reference position L(t2) of the posture detection plate 6 on the orthogonal coordinate axes O-xyz from the position orientation calculation unit 4. As the reference position L of the posture detection plate 6 is fixed to the xiphoid process, and the position vector OP0(t2) of the position P0(t2)ofthe xiphoid process (whose direction components are xP0(t2), yP0(t2), zP0(t2) on the orthogonal coordinate axes O-xyz) is the same as the OL(t2), the following formula 13 may be expressed.

[Formula 13]

$$OP_0(t2) = x_{P0}(t2)\mathbf{i} + y_{P0}(t2)\mathbf{j} + z_{P0}(t2)\mathbf{k} = OL(t2)$$

Moreover, the 3D guide image forming circuit 63 simultaneously obtains the rotating matrix T(t2) that indicatres the orientation of the posture detection plate 6 from the position orientation calculation unit 4. The rotating matrix T is used for correcting the change in each position of the sample points P0, P1, P2 and P3 caused by the change in the posture of the subject as described above. The process for the correction will be described later.

Thus, the 3D guide image forming circuit 63 has been able to obtain the respective direction components at the time t2 of the OPO(t2) and the OP2(t2) on the orthogonal coordinate axes O-xyz, and the rotating matrix T(t2).

The 3D guide image forming circuit 63 writes the respective direction components at the time t2 of the OP0(t2) and OP2(t2) on the orthogonal coordinate axes O-xyz, and the rotating matrix T(t2) into the volume memory 64 (step S41).

In the process executed from steps S37 to S41, the pylorus is set as the sample point on the body cavity surface. In the present embodiment, the same process may further be executed having the duodenal papilla set as the sample point. Accordingly, the operations corresponding to the aforementioned steps from S37 to S41 will be designated as steps S37' to S41' which are not shown in Fig. 14.

The operator inserts the rigid portion 21 and the flexible portion 22 into the body cavity of the subject, searches the sample point while observing the optical image such that the rigid portion 21 is moved to be adjacent to the sample point (duodenal papilla)(step S37').

Then the operator inserts the position detection probe 8 from the forceps end 52 while observing the optical image such that the tip protrudes from the protruding end 53. Then, the operator brings the tip of the position detection probe 8 into contact with the sample point (duodenal papilla) under the optical visual firled (step S38').

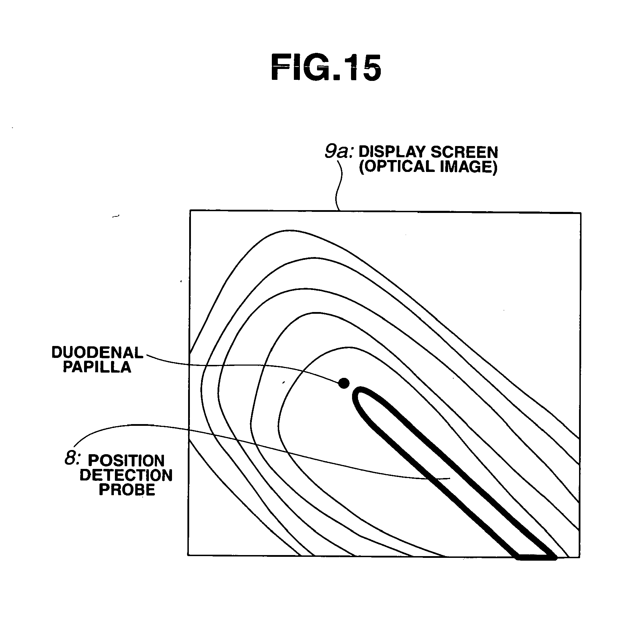

The aforementioned operation will be described referring to Fig. 15. Fig. 15 is a view showing the optical image on the display screen 9a when the position detection probe 8 is brought into contact with the duodenal papilla. Referring to Fig. 15, the tip of the position detection probe 8 is set to be positioned within the optical field range covered by the optical observation window 24 such that the duodenal papilla and the position detection probe 8 are shown on the optical image of the display screen 9a. The operator brings the tip of the position detection probe 8 into contact with the duodenal papilla while observing the optical image.

When the tip of the position detection probe 8 is brought into contact with the sample point (duodenal papilla), the operator presses the body cavity surface sample point designation key 12g on the keyboard 12 (step S39'). The time when the aforementioned operation is performed is defined as t3.

Then, the 3D guide image forming cirucit 63 loads the position/orientation data from the position orientation calculation unit 4 (step S40').

The 3D guide image forming circuit 63 obtains the respective direction components of the position vector OC (t3) at the position C(t3) of the receiving coil 42 at the tip of the position detection probe 8 on the orthogonal coordinate axes O-xyz from the position/orientation data.

As the position vector OP3(t3) of the position P3 of the duodenal papilla (whose direction components are xP3 (t3), yP3(t3), zP3(t3) on the orthogonal coordinate axes O-xyz) is the same as the OC(t3), the following formula 14 may be expressed.

[Formula 14]

$$\mathbf{OP}_3(t3) = x_{P3}(t3)\mathbf{i} + y_{P3}(t3)\mathbf{j} + z_{P3}(t3)\mathbf{k} = \mathbf{OC}(t3)$$

At this time, the 3D guide image forming circuit 63 simultaneously obtains the respective direction components of the position vector OL(t3) at the reference position L(t3) of the posture detection plate 6 on the orthogonal coordinate axes O-xyz from the position orientation calculation unit 4. As the reference position L of the posture detection plate 6 is fixed to the xiphoid process, and the position vector OPO(t3) of the position P0(t3) of the xiphoid process (whose direction components are xP0(t3), yP0(t3), zP0(t3) on the orthogonal coordinate axes O-xyz) is the same as the OL(t3), the following formula 15 may be expressed.

[Formula 15]

$$\mathbf{OP}_0(t3) = x_{P0}(t3)\mathbf{i} + y_{P0}(t3)\mathbf{j} + z_{P0}(t3)\mathbf{k} = \mathbf{OL}(t3)$$

Moreover, the 3D guide image forming circuit 63 simultaneously obtains the rotating matrix T(t3) that indicates the orientation of the posture detection plate 6 from the position orientation calculation unit 4. The rotating matrix T is used for correcting the change in each position of the respective sample points of P0, P1, P2 and P3 caused by the change in the posture of the subject. The process for the correction will be described later.

Thus, the 3D guide image forming circuit 63 has been able to obtain the respective direction components of the OP0(t3) and OP3(t3) on the orthogonal coordinate axes O-xyz, and the rotating matrix T(t3) at the time t3, respectively.

Next, the 3D guide image forming circuit 63 writes the respective direction components of the OP0(t3) and OP3(t3) on the orthogonal coordinate axes O-xyz, and the rotating matrix T(t3) at the time t3 into the volume memory 64 (step S41').

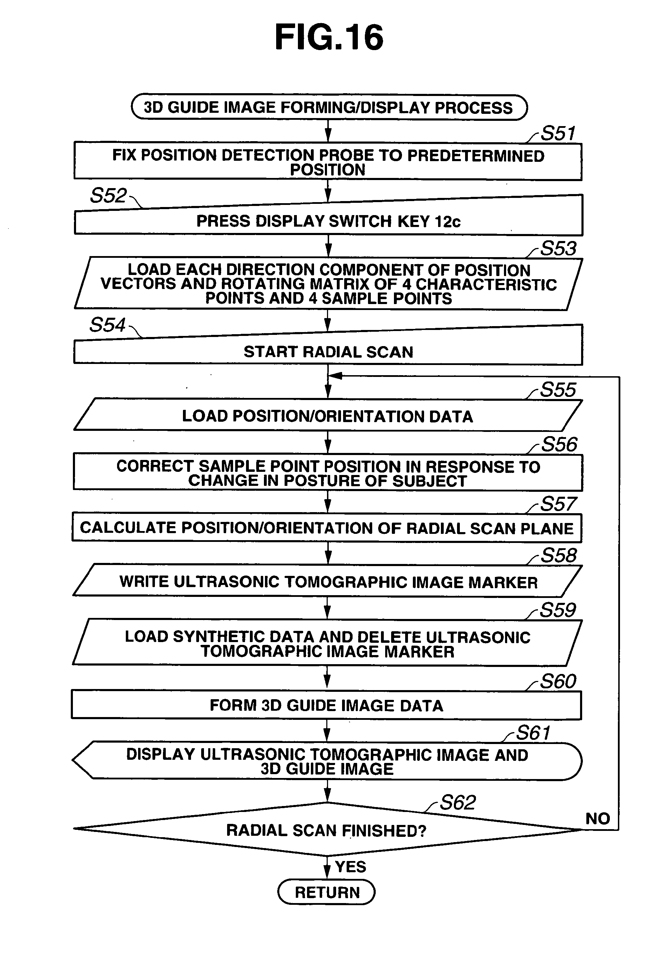

Fig. 16 is a flowchart showing the detail of the 3D gide image formation/display process executed in step S4 shown in Fig. 8.

Upon start of the routine, the operator makes the position of the forceps marker 44 of the position detection probe 8 coincided with the position of the open plane of the forceps end 52. At this time, the position of the tip of the position detection probe 8 is made coincided with the position of the open plane of the protruding end 53 to establish the predetermined positional relationship such that the receiving coil 42 is arranged considerably adjacent to the rotating center of the radial scan performed by the ultrasonic oschillator 31. Further, the operator rotates the postion detection probe 8 until the position of the 12 o'clock direction marker 45 at the probe side of the position detection probe 8 coincides with the position of the 12 o'clock direction marker 55 at the endoscope side disposed around the forceps end 52 of the operation portion 23. At this time, the vector Vc shown in Fig. 2 coincides with the vector V shown in Fig. 1, the vector Va shown in Fig. 2 coincides with the vector V3 shown in Fig. 1, and the vector Vb shown in Fig. 2 coincides with the vector V12 shown in Fig. 1, respectively. The operator fixes the position detection probe 8 so as not to move wihtin the forceps channel 51 (step S51).

The aforementioned fixing operation provides the contents of the position/orientation data as regarded below.

As the receiving coil 42 is fixed to the portion adjacent to the ultrasonic transducer 31, the positiosn vector OC (t) of the receiving coil 42 may be practically considered as the position vector at the rotating center of the ultrasonic transducer 31.

As the direction unit vector Vc that indicates the first winding axial direction of the receiving coil 42 coincides with the vector V as shown in Fig. 1, the Vc(t) may be practically regarded as the vector V that indicates the normal direction of the radial scan plane of the ultrasonic transducer 31, that is, the normal direction of the ultrasonic tomographic image data.

As the direction unit vector Vb(t) that indicates the second winding axial direction of the receiving coil 42 coincides with the vector V12 as shown in Fig. 1, the Vb(t) may be practically regarded as the vector V12 that indicates the 12 o'clock direction on the radial scan plane of the ultrasonic transducer 31.

The explanation will be made by defining the vectors V and V 12 as functions of time V(t), V12(t), and replacing the Vc(t) and Vb(t) with the V(t) and V12(t), respectively.

Subsequently, the control circuit 67 detects that the operator has pressed the display switch key 12c on the keyaboard 12, and allows the switch 68 of the display circuit 66 to be switched to the input temrinal 68c (step S52).

The 3D guide image forming cirucit 63 loads the respective direction components of the position vectors of four characteristic points P0', P1', P2' and P3' on the orthogonal coordinate axes O'-x'y'z' from the volume memory 64. Moreover, the 3D guide image forming circuit 63 loads the respective direction components of the four sample points P0, P1, P2 and P3 on the orthogonal coordinate axes O-xyz, the respective direction components of the position vectors OPO(tl), OPO(t2) and OPO(t3) of the xiphoid process at the position P0 on the orthogonal coordinate axes O-xyz at the time when the respective direction components of those sample points P0, P1, P2 and P3 are obtained, and the rotating matrixes T(t1), T(t2) and T(t3) from the volume memory 64 (step S53).

Thereafter, when the operator presses the scan control key 12h on the keyboard 12, the control circuit 67 detects the aforemeionted operation to allow the ultrasonic transducer 31 to start radial scan (step S54). In response to the radial scan, the ultrasonic tomographic image data are successively input to the mixing circuit 65 from the ultrasonic observation unti 3.

Every time when the ultrasonic transducer 31 performs the radial scan to allow the ultrasonic observation unit 3 to form the ultrasonic tomographic image data such that the ultrasonic tomographic image data are input to the mixing circuit 65 from the ultrasonic observation unit 3, the control circuit 67 outputs a command signal to the 3D guide image forming cirucit 63. The 3D guide image forming circuit 63 loads the position/orientation data from the position orientation calculation unit 4 upon reception of the command (step S55). The time when the aformentioned operation is performed is defined as ts.

The 3D guide image forming circuit 63 obtains the following data (1) to (5) from the loaded position/orientation data:

(1) the respective direction components of the position vector OC(ts) of the receiving coil 42 at the position C(ts) on the orthogonal coordinate axes O-xyz;
(2) the respective direction components of the position vector V(ts) indicating the first winding axial direction of the receiving coil 42 on the orthogonal coordinate axes O-xyz;
(3) the respective direction components of the direction vector V12(ts) indicating the second winding axial directionof the receiving coil 42 on the orthogonal cordinate axis O-xyz;
(4) the respective direction components of the position vector OL(ts) of the posture detection plate 6 at the reference position L(ts) on the orthogonal coordinate axes O-xyz; and
(5) the 3 x 3 rotating matrix T(ts) indicdating the orientation of the posture detection plate 6.

The OC(ts), V(ts) and V 12(ts) are obtained in order to allow the 3D guide image forming circuit 63 to correct the position and direction of the radial scan plane correctly coincided with the current position and direction constnatly as described later.

The OL(ts) and T(ts) are obtained to constantly allow the 3D guide image forming circuit 63 to accurately correct the current positions of the sample points P0, P1, P2 and P3 which are moved in accordance with the change in the posture of the subject as described later.

The 3D guide image forming circuit 63 corrects the current positions of the sample points P0, P1, P2 and P3 at the time ts moved in accordance with the change in the posture of the subject using the following formula 16 (step S56). The formula 16 is established on the assumption that the positional relationships among the P0, P1, P2 and P3 are kept unchanged irrespective of time passage without causing the subject's body to expand and distort.

[Formula 16]

$$\begin{pmatrix} x_{Pk}(ts) \\ y_{Pk}(ts) \\ z_{Pk}(ts) \end{pmatrix} = \begin{pmatrix} x_{P0}(ts) \\ y_{P0}(ts) \\ z_{P0}(ts) \end{pmatrix} + {}^t T(ts)T(ta) \begin{pmatrix} x_{Pk}(ta) - x_{P0}(ta) \\ y_{Pk}(ta) - y_{P0}(ta) \\ z_{Pk}(ta) - z_{P0}(ta) \end{pmatrix}$$

where the suffix k denotes any one of 1, 2 and 3, and the time ta denotes an arbitrarily set value prior to the time ts. The superscript "t" to the left of the matrix T denotes the transpose to indicate the transposed matrix of T. As the T is the orthogonal matrix, the transposed matrix of T is equivalent to the inverse matrix of T. The process for deriving the aforemenitoned formula 16 will not be described in detail, but briefly explained hereinafter. That is, the respective direction components of the sample point Pk on the orthogonal coordinate axes O-xyz at the time ts is obtained by adding the respective direction components of the sample point P0 at the time ts on the orthogonal coordinate axes O-xyz to the respective direction components of the vector P0 Pk at the time ts on the orthogonal coordinate axes O-xyz. The respective direction components of the vectors P0 Pk at the time ts on the orthogonal coordinate axes O-xyz are obtained by converting the respective direction components of the vectors P0 Pk at the time ta on the orthogonal coordinate axes O-xyz into those at the time ta on the orthogonal coordinate axes O"-x"y"z" so as to be further converted into those at the time ts on the orthogonal coordinate axes O-xyz. The formula 16, thus, is derived from the aforementioned operation.

The 3D guide image forming circuit 63 is allowed to accurately correct the position vectors of the sample points P0, P1, P2 and P3, and the respective direction components on the orthogonal coordinate axes O-xyz thereof at the time ts as represented by the following formula using the formula 16 based on the respective direction components of the position vectors of the four characteristic points P0', P 1 ', P2' and P3' on the orthogonal coordinate axes O'-x'y'z', the respective direction components of the four sample points P0, P1, P2 and P3 on the orthogonal coordinate axes O-xyz, the respective direction components of the position vectors OPO(tl), OP0(t2) and OP0(t3) of the xiphoid process at the position P0 at the time when the respective direction components of those sample points P0, P1, P2 and P3 are obtained, and the rotating matrixes T(t1), T(t2) and T(t3), which are loaded from the volume memory 64 in step 553.

The 3D guide image forming circuit 63 is allowed to accurately calculate the position vectors OP0, OP1, OP2 and OP3 of the sample points P0, P1, P2 and P3 at the time ts, and the respective direction components on the orthogonal coordinate axes O-xyz thereof using the respective direction components of the position vector OL of the posture detection plate 6 at the reference position L at the time ts when they are obtained from the position orientation calculation unit 4, and the 3 x 3 rotating matrix T indicating the orientation of the posture detection plate 6 at the time ts even if the posture of the subject changes.

As the position of the sample point P0 (xiphoid process) at the time ts is the same as the reference position L of the posture detection plate 6 at the time ts, the correction of the sample point P0 (xiphoid process) is performed as shown by the following formula 17.

[Formula 17]

$$\mathbf{OP}_0(ts) = x_{P0}(ts)\mathbf{i} + y_{P0}(ts)\mathbf{j} + z_{P0}(ts)\mathbf{k} = \mathbf{OL}(ts)$$

Next, based on the formula 16, the correction of the sample point P1 (right end of pelvis) is performed as shown by the following formulae 18 and 19.

[Formula 18]

$$\begin{pmatrix} x_{P1}(ts) \\ y_{P1}(ts) \\ z_{P1}(ts) \end{pmatrix} = \begin{pmatrix} x_{P0}(ts) \\ y_{P0}(ts) \\ z_{P0}(ts) \end{pmatrix} + {}^{t}T(ts)T(t1)\begin{pmatrix} x_{P1}(t1) - x_{P0}(t1) \\ y_{P1}(t1) - y_{P0}(t1) \\ z_{P1}(t1) - z_{P0}(t1) \end{pmatrix}$$

[Formula 19]

$$\mathbf{OP}_1(ts) = x_{P1}(ts)\mathbf{i} + y_{P1}(ts)\mathbf{j} + z_{P1}(ts)\mathbf{k}$$

Moreover, based on the formula 16, the correction of the sample point P2 (pylorus) is performed as shown by the following formulae 20 and 21.

[Formula 20]

$$\begin{pmatrix} x_{P2}(ts) \\ y_{P2}(ts) \\ z_{P2}(ts) \end{pmatrix} = \begin{pmatrix} x_{P0}(ts) \\ y_{P0}(ts) \\ z_{P0}(ts) \end{pmatrix} + {}^{t}T(ts)T(t2) \begin{pmatrix} x_{P2}(t2) - x_{P0}(t2) \\ y_{P2}(t2) - y_{P0}(t2) \\ z_{P2}(t2) - z_{P0}(t2) \end{pmatrix}$$

[Formula 21]

$$\mathbf{OP}_2(ts) = x_{P2}(ts)\mathbf{i} + y_{P2}(ts)\mathbf{j} + z_{P2}(ts)\mathbf{k}$$

And, based on the formula 16, the correction of the sample P3 (duodenal papilla) is performed as shown by the following formulae 22 and 23.

[Formula 22]

$$\begin{pmatrix} x_{P3}(ts) \\ y_{P3}(ts) \\ z_{P3}(ts) \end{pmatrix} = \begin{pmatrix} x_{P0}(ts) \\ y_{P0}(ts) \\ z_{P0}(ts) \end{pmatrix} + {}^{t}T(ts)T(t3) \begin{pmatrix} x_{P3}(t3) - x_{P0}(t3) \\ y_{P3}(t3) - y_{P0}(t3) \\ z_{P3}(t3) - z_{P0}(t3) \end{pmatrix}$$

[Formula 23]

$$\mathbf{OP}_3(ts) = x_{P3}(ts)\mathbf{i} + y_{P3}(ts)\mathbf{j} + z_{P3}(ts)\mathbf{k}$$

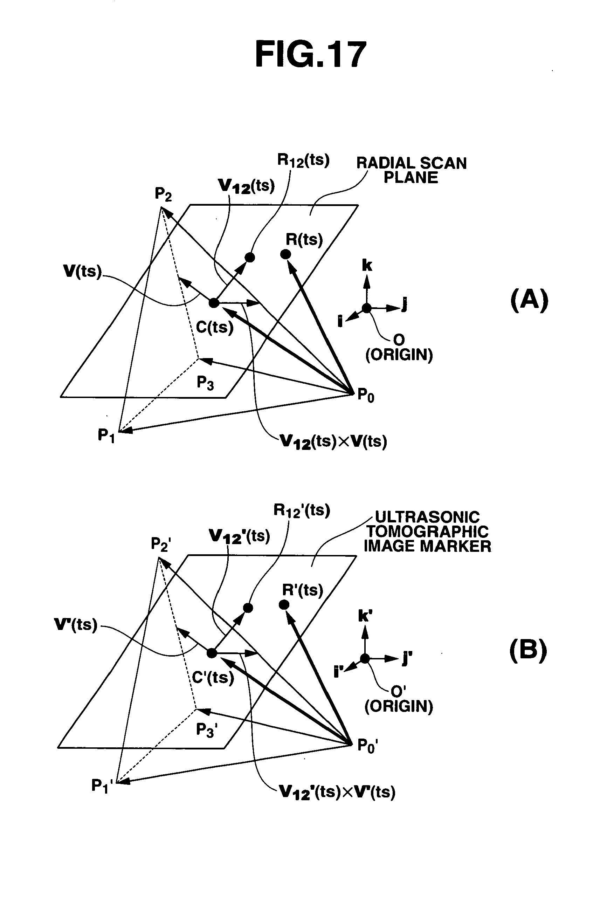

Thereafter, the 3D guide image forming cirucit 63 calculates the position and orientation of the radial scan plane (step S57). The aforementioned process will be described referring to Fig. 17. Fig. 17 shows the relationship between the sample points and the radial scan plane, and the relationhip between the characteirstic points and the ultrasonic tomographic image marker, respectively. Fig. 17(A) is a view representing the relationship between the sample points and the radial scan plane. Fig. 17(B) is a view reprenting the relationship between the characteirstic points and the ultrasonic tomographic image marker.

The 3D guide image forming cirucit 63 calculates the position and orientation of the index (referred to as the ultrasonic tomographic image marker) that indicates the ultrasonic tomographic image in the voxel space as well as extracts the data to be extracted.

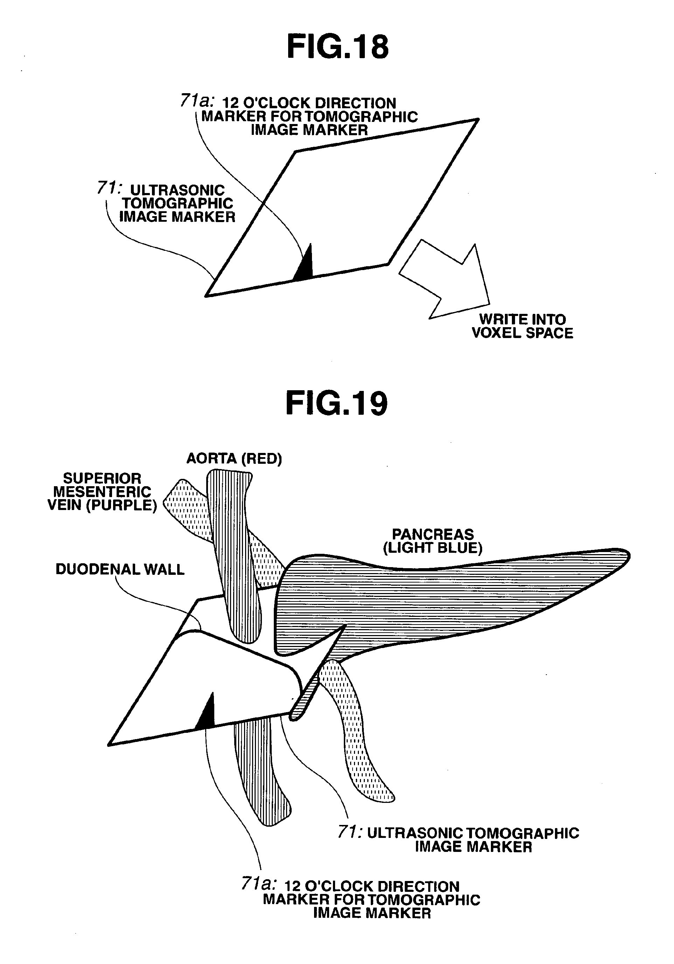

Fig. 18 is a view showing the ultrasonic tomographic image marker 71.

The ultrasonic tomographic image marker 71 is positioned within the voxel space having its position and orientation anatomically coincided with those of the ultrasonic tomographic image data output from the ultrasonic observation unit 3 with respect to the radial scan at one rotation performed by the ultrasonic transducer 31.

Referring to Fig. 17(B), the center of the ultrasonic tomographic image marker 71 is defined as the point C'(ts), the normal vector of the ultrasonic tomographic image marker 71 is definged as V'(ts), and the 12 o'clock direction vector is defined as V12'(ts), respectively. The 3 o'clock direction vector is defined as V12'(ts) x V'(ts) accordingly (as the exterior product ofV12'(ts) and V'(ts)). The ultrasonic tomographic image marker 71 becomes a set of points R'(ts) at which the position vector satisfies the following formula 24 as shown in Fig. 17(B) (the point R'(ts) corresponds with the arbitrary point R(ts) on the radial scan plane).

[Formula 24]

$$\mathbf{O'R'}(ts) = \mathbf{O'C'}(ts) + X'\left\{\mathbf{V_{12}'}(ts) \times \mathbf{V'}(ts)\right\} + Y'\mathbf{V_{12}'}(ts)$$

In the formula 24, the term X' denotes the distance between the point R'(ts) and the point C'(ts) in 3 o'clock direction, and Y' denotes the distance between the point R'(ts) and the point C'(ts) in 12 o'clock direction.

In the description hereinafter, fundamentals of (a) correlation between the arbitrary point on the plane of the ultrasonic tomographic image data and the point on the ultrasonic tomographic image marker 71, and (b) the process for calculating the center position, normal direction and 12 o'clock direction of the ultrasonic tomographic image marker 71 as the positin and orientation thereof will be explained.

To describe in advance, the 3D guide image forming cirucit 63 calculates the position vector O'C'(ts) at the center position C'(ts) and the respective direction components on the rothogonal coordinate axes O'-x'y'z' therof using the formulae 41 and 42 based on the following fundamental. And, the 3D guide image forming circuit 63 calculates the 12 o'clock direction vector V12'(ts) of the ultrasonic tomograhpic image marker 71 and the respective direction components on the orthogonal coordinate axes O'-x'y'z' thereof using the formula 48 or the formulae 52 and 53 based on the fundamental to be described below. Moreover, the 3D guide image forming cirucit 63 calculates the normal vector V'(ts) of the ultrasonic tomographic image marker 71 and the respective direction components on the orthogonal coordinate axes O'-x'y'z' thereof using the formulae 65 and 66 based on the fundamental to be described below.

First, the fundamental of (a) the correlation between arbitrary points on the plane of the ultrasonic tomographic image data and the points on the ultrasonic tomographic image marker 71 will be described.

Assuming that the point R(ts) on the orthogonal coordinate axes O-xyz is an arbitrary point on the radial scan plane, the reference position L of the posture detection plate 6, that is, the position vector P0R(ts) between the points P0 and R(ts) may be expressed as shown by the following formula 25 using the appropriate real numbers of a, b and c.

[Formula 25]

$$\mathbf{P_0R}(ts) = a\mathbf{P_0P_1}(ts) + b\mathbf{P_0P_2}(ts) + c\mathbf{P_0P_3}(ts)$$

In the formula 25, all the vectors are considered as the function of time.

Meanwhile, the characteirstic points P0', P1', P2' and P3' on the reference image data 61a are correlated with the sample poitns P0, P1, P2 and P3 at anatomially the same positions, respectively. Generally, the human body has substnantially the same anatomical structure. In the case where the point R(ts) is at the specific position with respect to the triangular pyramid defined by P0, P1, P2 and P3 having the sample point as the apex, the point R'(ts) at the corresponding position with respect to the triangular pyramid defined by P0', P 1', P2' and P3' having the characteirstic point as apex on the reference image data 61a is assumed to correspond with the point that is the same as the point R(ts) on the anatomically same organ, or same tissue. Based on the assumption, the point anatomically corresponding to the point R(ts) is determined as the point R'(ts) on the orthogonal coordinate axes O'-x'y'z' that can be similarly expressed as shown in the following formula 26 using the real numbers of a, b and c.

[Formula 26]

$$\mathbf{P_0'R'}(ts) = a\mathbf{P_0'P_1'} + b\mathbf{P_0'P_2'} + c\mathbf{P_0'P_3'}$$

Assuming that the respective direciton components of the position vector OR(ts) of the point R(ts) on the orthogonal coordinate axes O-xyz are defined as xR(ts), yR(ts) and zR(ts), and the respective direction components of the position vector O'R'(ts) of the poitn R'(ts) on the orthogonal coordinate axes O'-x'y'z' are defined as xR'(ts), yR'(ts) and zR'(ts), respectively, the following formulae 27 and 28 are established.

[Formula 27]

$$\mathbf{OR}(ts) = x_R(ts)\mathbf{i} + y_R(ts)\mathbf{j} + z_R(ts)\mathbf{k}$$

[Formula 28]

$$\mathbf{O'R'}(ts) = x_R{}'(ts)\mathbf{i'} + y_R{}'(ts)\mathbf{j'} + z_R{}'(ts)\mathbf{k'}$$

Hereinbelow, based on the formulae which have been described so far, the position vector O'R' of the point R' (ts) anatomically corresponding to the arbitrary point R(ts) on the radial scan plane on the orthogonal coordinate axes O-xyz, and the respective direction components xR'(ts), yR'(ts) and zR'(ts) on the orthogonal coordinate axes O'-x'y'z' thereof are obtained.

The following formula 29 is established by the formula 25.

[Formula 29]

$$\mathbf{OR}(ts) - \mathbf{OP_0}(ts)$$
$$= a\{\mathbf{OP_1}(ts) - \mathbf{OP_0}(ts)\} + b\{\mathbf{OP_2}(ts) - \mathbf{OP_0}(ts)\} + c\{\mathbf{OP_3}(ts) - \mathbf{OP_0}(ts)\}$$

The following formula 30 is established by the formulae 27, 29 and 17 to 23.

[Formula 30]

$$
\begin{pmatrix} x_R(ts) \\ y_R(ts) \\ z_R(ts) \end{pmatrix} - \begin{pmatrix} x_{P0}(ts) \\ y_{P0}(ts) \\ z_{P0}(ts) \end{pmatrix} = \begin{pmatrix} x_{P1}(ts) - x_{P0}(ts) & x_{P2}(ts) - x_{P0}(ts) & x_{P3}(ts) - x_{P0}(ts) \\ y_{P1}(ts) - y_{P0}(ts) & y_{P2}(ts) - y_{P0}(ts) & y_{P3}(ts) - y_{P0}(ts) \\ z_{P1}(ts) - z_{P0}(ts) & z_{P2}(ts) - z_{P0}(ts) & z_{P3}(ts) - z_{P0}(ts) \end{pmatrix} \begin{pmatrix} a \\ b \\ c \end{pmatrix}
$$

The 3 x 3 matrix Q(ts) is defined as the following formula 31 for simplifying the expression of the subsequent formulae.

[Formula 31]

$$
\mathbf{Q}(ts) = \begin{pmatrix} x_{P1}(ts) - x_{P0}(ts) & x_{P2}(ts) - x_{P0}(ts) & x_{P3}(ts) - x_{P0}(ts) \\ y_{P1}(ts) - y_{P0}(ts) & y_{P2}(ts) - y_{P0}(ts) & y_{P3}(ts) - y_{P0}(ts) \\ z_{P1}(ts) - z_{P0}(ts) & z_{P2}(ts) - z_{P0}(ts) & z_{P3}(ts) - z_{P0}(ts) \end{pmatrix}
$$

The formula 30 may be expressed as the following formula 32 using the formula 31.

[Formula 32]

$$\begin{pmatrix} x_R(ts) \\ y_R(ts) \\ z_R(ts) \end{pmatrix} - \begin{pmatrix} x_{P0}(ts) \\ y_{P0}(ts) \\ z_{P0}(ts) \end{pmatrix} = Q(ts) \begin{pmatrix} a \\ b \\ c \end{pmatrix}$$

The following formula 33 for obtaining the values of a, b and c is derived from multiplying the left term of the formula 32 by the inverse matrix of the matrix Q(ts).

[Formula 33]

$$\begin{pmatrix} a \\ b \\ c \end{pmatrix} = Q(ts)^{-1} \left\{ \begin{pmatrix} x_R(ts) \\ y_R(ts) \\ z_R(ts) \end{pmatrix} - \begin{pmatrix} x_{P0}(ts) \\ y_{P0}(ts) \\ z_{P0}(ts) \end{pmatrix} \right\}$$

Meanwhile, the following formula 34 like the formula 29 is derived from the formula 26.

[Formula 34]

$$\mathbf{O'R'}(ts) - \mathbf{O'P_0}' = a\left(\mathbf{O'P_1}' - \mathbf{O'P_0}'\right) + b\left(\mathbf{O'P_2}' - \mathbf{O'P_0}'\right) + c\left(\mathbf{O'P_3}' - \mathbf{O'P_0}'\right)$$

Likewise the formula 30 derived from the formulae 27, 29, and 17 to 23, the following formula 35 is derived from the formulae 28, 34, and 6 to 9.

[Formula 35]

$$\begin{pmatrix} x_R'(ts) \\ y_R'(ts) \\ z_R'(ts) \end{pmatrix} - \begin{pmatrix} x_{P0}' \\ y_{P0}' \\ z_{P0}' \end{pmatrix} = \begin{pmatrix} x_{P1}' - x_{P0}' & x_{P2}' - x_{P0}' & x_{P3}' - x_{P0}' \\ y_{P1}' - y_{P0}' & y_{P2}' - y_{P0}' & y_{P3}' - y_{P0}' \\ z_{P1}' - z_{P0}' & z_{P2}' - z_{P0}' & z_{P3}' - z_{P0}' \end{pmatrix} \begin{pmatrix} a \\ b \\ c \end{pmatrix}$$

The 3 x 3 matrix Q' is also defined as the following formula 36 for simplifying the expression of the subsequent formulae.

[Formula 36]

$$Q' = \begin{pmatrix} x_{P1}' - x_{P0}' & x_{P2}' - x_{P0}' & x_{P3}' - x_{P0}' \\ y_{P1}' - y_{P0}' & y_{P2}' - y_{P0}' & y_{P3}' - y_{P0}' \\ z_{P1}' - z_{P0}' & z_{P2}' - z_{P0}' & z_{P3}' - z_{P0}' \end{pmatrix}$$

The formula 35 may be expressed as the following formula 37 using the expression of the above formula 36.

[Formula 37]

$$\begin{pmatrix} x_R{}'(ts) \\ y_R{}'(ts) \\ z_R{}'(ts) \end{pmatrix} - \begin{pmatrix} x_{P0}{}' \\ y_{P0}{}' \\ z_{P0}{}' \end{pmatrix} = \mathbf{Q}' \begin{pmatrix} a \\ b \\ c \end{pmatrix}$$

As those values of a, b and c are obtained by the formula 33, they are assigned to the formula 37 to obtain the following formula 38.

[Formula 38]

$$\begin{pmatrix} x_R{}'(ts) \\ y_R{}'(ts) \\ z_R{}'(ts) \end{pmatrix} - \begin{pmatrix} x_{P0}{}' \\ y_{P0}{}' \\ z_{P0}{}' \end{pmatrix} = \mathbf{Q}'\mathbf{Q}(ts)^{-1} \left\{ \begin{pmatrix} x_R(ts) \\ y_R(ts) \\ z_R(ts) \end{pmatrix} - \begin{pmatrix} x_{P0}(ts) \\ y_{P0}(ts) \\ z_{P0}(ts) \end{pmatrix} \right\}$$

Accordingly, the following formula 39 is obtained.

[Formula 39]

$$\begin{pmatrix} x_R{}'(ts) \\ y_R{}'(ts) \\ z_R{}'(ts) \end{pmatrix} = \begin{pmatrix} x_{P0}{}' \\ y_{P0}{}' \\ z_{P0}{}' \end{pmatrix} + \mathbf{Q}'\mathbf{Q}(ts)^{-1} \left\{ \begin{pmatrix} x_R(ts) \\ y_R(ts) \\ z_R(ts) \end{pmatrix} - \begin{pmatrix} x_{P0}(ts) \\ y_{P0}(ts) \\ z_{P0}(ts) \end{pmatrix} \right\}$$

The position vectors O'R'(ts) of the point R'(ts) anatomically corresponding to the arbitrary point R(ts) on the radial scan plane on the orthogonal coordinate axes O-xyz, and the respective direction components xR'(ts), yR'(ts) and zR'(ts) on the orthogonal coordinate axes O'-x'y'z' may be obtained using the formulae 28 and 39. The ultrasonic tomograhpic image marker 71 may be determined as the set of the points R'(ts) with respect to the arbitrary point R(ts) on the radial scan plane, which are derived from the formulae 28 and 39 using the respective direction components of the four characteirstic points and the four sample points and the rotating matrixes loaded by the 3D guide image forming circuit 63 from the volume memory 64 in step 553.

Next, the fundamental of (b) process for calculating the center position, normal direction and 12 o'clock direction of the ultrasonic tomographic image marker 71 will be described.

The correlation between the arbitrary points R(ts) on the radial scan plane on the orthogonal coordinate axes O-xyz and the anatomically corresponding point R'(ts) on the orthogonal coordinate axes O'-x'y'z' is derived from the formula 39.

The process for calculating the center position C'(ts), the normal vector V'(ts) and the 12 o'clock direction vector V12' (ts) of the ultrasonic tomographic image marker 71 will be described hereinafter.

(b-1) Calculation of center position C'(ts) of the ultrasonic tomographic image marker 71

The point anatomically corresponding to the position C(ts) of the receiving coil 42 is defined as C'(ts). The point C(ts) is the rotating center of the ultrasonic transducer 31, and accordingly, it becomes the center on the radial scan plane. Therefore, the point C'(ts) becomes the center of the ultrasonic tomographic image marker 71.

Assuming that the respective direction components of the OC(ts) on the orthogonal coordinate axes O-xyz are defined as xC(ts), yC(ts) and zC(ts), the following formula 40 is established.

[Formula 40]

$$\mathbf{OC}(ts) = x_C(ts)\mathbf{i} + y_C(ts)\mathbf{j} + z_C(ts)\mathbf{k}$$

Assuming that the respective direction components of the O'C'(ts) on the orthogonal .coordinate axes O'-x'y'z' are defiend as xC'(ts), yC'(ts) and zC'(ts), the following formula 41 is established.

[Formula 41]

$$\mathbf{O'C'}(ts) = x_C{}'(ts)\mathbf{i'} + y_C{}'(ts)\mathbf{j'} + z_C{}'(ts)\mathbf{k'}$$

Assuming that the points R(ts) and R'(ts) are detemrined as the poitns C(ts) and C'(ts), respectively, the following formula 42 is derived from the formula 39 where the point R(ts) is the arbitrary point on the radial scan plane in the above-described formula 39.

[Formula 42]

$$\begin{pmatrix} x_C{}'(ts) \\ y_C{}'(ts) \\ z_C{}'(ts) \end{pmatrix} = \begin{pmatrix} x_{P0}{}' \\ y_{P0}{}' \\ z_{P0}{}' \end{pmatrix} + \mathbf{Q'Q}(ts)^{-1} \left\{ \begin{pmatrix} x_C(ts) \\ y_C(ts) \\ z_C(ts) \end{pmatrix} - \begin{pmatrix} x_{P0}(ts) \\ y_{P0}(ts) \\ z_{P0}(ts) \end{pmatrix} \right\}$$

Thus, the center rpoisiton C'(ts) of the ultrasonic tomographic image marker 71 on the reference image data 61 a may be obtained using the formulae 41 and 42. (b-2): Calculation of 12 o'clock direction vector V 12'(ts) of the ultrasonic tomographic image marker 71

It is assumed that the point on the radial scan plane at the unit distance from the center C(ts) on the plane toward the 12 o'clock direction is R12(ts) (see Fig. 17(A), and the point anatomically corresponding to the point R12(ts) is defined as R12'(ts).

Assuming that the respective direction components of the OR12(ts) on the orthogonal coordinate axes O-xyz are defined as xR12(ts), yR12(ts) and zR12(ts), the following formula 43 is established.

[Formula 43]

$$\mathbf{OR}_{12}(ts) = x_{R12}(ts)\mathbf{i} + y_{R12}(ts)\mathbf{j} + z_{R12}(ts)\mathbf{k}$$

Assuming that the respective direction components of the O'R12'(ts) on the orthogonal coordinate axes O'-x'y'z' are defined as xR12'(ts), yR12'(ts) and zR12'(ts), the following formula 44 is established.

[Formula 44]

$$\mathbf{O'R}_{12}{}'(ts) = x_{R12}{}'(ts)\mathbf{i'} + y_{R12}{}'(ts)\mathbf{j'} + z_{R12}{}'(ts)\mathbf{k'}$$

Assuming that the point R(ts) and R'(ts) are determined as the points R12(ts) and R12'(ts), respectively, the following formula 45 is derived from the formula 39 where the point R(ts) is an arbitrary point on the radial scan plane.

[Formula 45]

$$\begin{pmatrix} x_{R12}{}'(ts) \\ y_{R12}{}'(ts) \\ z_{R12}{}'(ts) \end{pmatrix} = \begin{pmatrix} x_{P0}{}' \\ y_{P0}{}' \\ z_{P0}{}' \end{pmatrix} + \mathbf{Q'Q}(ts)^{-1} \left\{ \begin{pmatrix} x_{R12}(ts) \\ y_{R12}(ts) \\ z_{R12}(ts) \end{pmatrix} - \begin{pmatrix} x_{P0}(ts) \\ y_{P0}(ts) \\ z_{P0}(ts) \end{pmatrix} \right\}$$

In this way, the point R12'(ts) anatomically corresponding to the point R12(ts) at the unit distance from the center C(ts) on the radial scan plane toward 12 o'clock direction may be derived from the formulae 44 and 45.
If the 12 o'clock direction vector V12(ts) of the ultrasonic tomographic image is used as the position vector of the point R12(ts), the following formula 46 is established.

[Formula 46]

$$\mathbf{OR}_{12}(ts) = \mathbf{OC}(ts) + \mathbf{V}_{12}(ts)$$

The formula 46 may be rewritten as the following formula 47.

[Formula 47]

$$\mathbf{V}_{12}(ts) = \mathbf{OR}_{12}(ts) - \mathbf{OC}(ts)$$

Therefore, it is obvious that the direction of the O'R12'(ts) minus O'C'(ts) is set to the direction of the 12 o'clock direction vector V 12'(ts) of the ultrasonic tomographic image marker 71 based on the correlation with the formula 47. Accordingly, the 12 o'clock direction vector V 12'(ts) of the ultrasonic tomographic image marker 71 may be obtained by normalizing the vector to have the unit length as shown by the following formula 48.

[Formula 48]

$$\mathbf{V}_{12}{}'(ts) = \frac{\mathbf{O'R}_{12}{}'(ts) - \mathbf{O'C}{}'(ts)}{\left| \mathbf{O'R}_{12}{}'(ts) - \mathbf{O'C}{}'(ts) \right|}$$

As the O'C'(ts) and O'12'(ts) have been already obtained by the formulae 41, 42 and 44, 45, respectively, the V 12'(ts) may be derived from the formula 48.
The process for obtaining the V 12'(ts) more clearly will be described hereinafter.
The following formula 49 is established by performing the subtraction between the formulae 42 and 45.

[Formula 49]

$$\begin{pmatrix} x_{R12}{}'(ts) \\ y_{R12}{}'(ts) \\ z_{R12}{}'(ts) \end{pmatrix} - \begin{pmatrix} x_C{}'(ts) \\ y_C{}'(ts) \\ z_C{}'(ts) \end{pmatrix} = \mathbf{Q'Q}(ts)^{-1} \left\{ \begin{pmatrix} x_{R12}(ts) \\ y_{R12}(ts) \\ z_{R12}(ts) \end{pmatrix} - \begin{pmatrix} x_C(ts) \\ y_C(ts) \\ z_C(ts) \end{pmatrix} \right\}$$

The left side of the formula 49 denotes the respective direction components of the O'R12'(ts)-O'C'(ts) on the orthogonal coordinate axes O'-x'y'z'. The respective direction components of the OR12(ts)-OC(ts) on the orthogonal coordinate axes O-xyz are in { } of the right side of the formula 49. The respective direction components of the V12(ts) on the orthogonal coordinate axes O-xyz are in { } of the right side of the formula 49 in reference to the formula 47. The 3D guide image forming circuit 63 obtains the respective direction components from the position orientation calculation unit 4 in step S55.

Assuming that the respective direction components of the V12(ts) on the orthogonal coordinate axes O-xyz are defined as xV 12(ts), yV12(ts) and zV 12(ts), the following formulae 50 and 51 are derived from the formula 49.

[Formula 50]

$$\mathbf{V}_{12}(ts) = x_{V12}(ts)\mathbf{i} + y_{V12}(ts)\mathbf{j} + z_{V12}(ts)\mathbf{k}$$

[Formula 51]

$$\begin{pmatrix} x_{R12}{}'(ts) \\ y_{R12}{}'(ts) \\ z_{R12}{}'(ts) \end{pmatrix} - \begin{pmatrix} x_{C}{}'(ts) \\ y_{C}{}'(ts) \\ z_{C}{}'(ts) \end{pmatrix} = \mathbf{Q}'\mathbf{Q}(ts)^{-1} \begin{pmatrix} x_{V12}(ts) \\ y_{V12}(ts) \\ z_{V12}(ts) \end{pmatrix}$$

The 12 o'clock direction vector V12'(ts) of the ultrasonic tomographic image marker 71 may be obtained as indicated by the following formulae 52 and 53 by normalizing the formula 51 in the same way as in the case of the formula 48.

[Formula 52]

$$\mathbf{V}_{12}{}'(ts) = x_{V12}{}'(ts)\mathbf{i}' + y_{V12}{}'(ts)\mathbf{j}' + z_{V12}{}'(ts)\mathbf{k}'$$

[Formula 53]

$$\begin{pmatrix} x_{V12}{}'(ts) \\ y_{V12}{}'(ts) \\ z_{V12}{}'(ts) \end{pmatrix} = \frac{\mathbf{Q}'\mathbf{Q}(ts)^{-1} \begin{pmatrix} x_{V12}(ts) \\ y_{V12}(ts) \\ z_{V12}(ts) \end{pmatrix}}{\left| \mathbf{Q}'\mathbf{Q}(ts)^{-1} \begin{pmatrix} x_{V12}(ts) \\ y_{V12}(ts) \\ z_{V12}(ts) \end{pmatrix} \right|}$$

where the respective direction components of the V 1 2'(ts) on the orthogonal coordinate axes O'-x'y'z' are defined as xV12'(ts), yV12'(ts) and zV12'(ts), respectively.

(b-3): Calculation of normal vector V'(ts) of the ultrasonic tomographic image marker 71

Assuming that the normal vector of the ultrasonic tomographic image marker 71 is defined as V'(ts), the V'(ts) becomes orthogonal to the arbitrary vector on the ultrasonic tomographic image marker 71. The calculation may be performed by searching the aforemeinoted vector.

The points R1'(ts) and R2'(ts) are defined as the arbitrary points on the ultrasonic tomographic image marker 71.

The points anatomically corresponding to the aforemeitoned points R1'(ts) and R2'(ts) may be defined as points R1(ts) and R2(ts), respectively. Assuming that the respective direction components of the point R1 (ts) on the orthogonal coordinate axes O-xyz are defined as xR1(ts), yR 1 (ts) and zR1(ts), and the respective direction components of the point R2(ts) on the orthogonal coordinate axes O-xyz are defined as xR2(ts), yR2(ts) and zR2(ts), the following formulae 54 and 55 are established.

[Formula 54]

$$\mathbf{OR}_1(ts) = x_{R1}(ts)\mathbf{i} + y_{R1}(ts)\mathbf{j} + z_{R1}(ts)\mathbf{k}$$

[Formula 55]

$$\mathbf{OR}_2(ts) = x_{R2}(ts)\mathbf{i} + y_{R2}(ts)\mathbf{j} + z_{R2}(ts)\mathbf{k}$$

Meanwhile, assuming that the respective direction components of the point R1'(ts) on the orthogonal coordinate axes O'-x'y'z' are defined as xR1'(ts), yR1'(ts) and zR1'(ts), and the respective direciton components of the point R2' (ts) on the orthogonal coordinate axes O'-x'y'z' are defined as xR2'(ts), yR2'(ts) and zR2'(ts), the following formulae 56 and 57 are established.

[Formula 56]

$$\mathbf{OR}_1{}'(ts) = x_{R1}{}'(ts)\mathbf{i}' + y_{R1}{}'(ts)\mathbf{j}' + z_{R1}{}'(ts)\mathbf{k}'$$

[Formula 57]

$$\mathbf{OR}_2{}'(ts) = x_{R2}{}'(ts)\mathbf{i}' + y_{R2}{}'(ts)\mathbf{j}' + z_{R2}{}'(ts)\mathbf{k}'$$

Based on the formula 39, the relationship between the points R1(ts) and R1'(ts) and the relationship between the points R2(ts) and R2'(ts) are expressed by the following formulae 58 and 59, respectively.

[Formula 58]

$$\begin{pmatrix} x_{R1}{}'(ts) \\ y_{R1}{}'(ts) \\ z_{R1}{}'(ts) \end{pmatrix} = \begin{pmatrix} x_{P0}{}' \\ y_{P0}{}' \\ z_{P0}{}' \end{pmatrix} + \mathbf{Q}'\mathbf{Q}(ts)^{-1} \left\{ \begin{pmatrix} x_{R1}(ts) \\ y_{R1}(ts) \\ z_{R1}(ts) \end{pmatrix} - \begin{pmatrix} x_{P0}(ts) \\ y_{P0}(ts) \\ z_{P0}(ts) \end{pmatrix} \right\}$$

[Formula 59]

$$\begin{pmatrix} x_{R2}'(ts) \\ y_{R2}'(ts) \\ z_{R2}'(ts) \end{pmatrix} = \begin{pmatrix} x_{P0}' \\ y_{P0}' \\ z_{P0}' \end{pmatrix} + \mathbf{Q}'\mathbf{Q}(ts)^{-1} \left\{ \begin{pmatrix} x_{R2}(ts) \\ y_{R2}(ts) \\ z_{R2}(ts) \end{pmatrix} - \begin{pmatrix} x_{P0}(ts) \\ y_{P0}(ts) \\ z_{P0}(ts) \end{pmatrix} \right\}$$

The following formula 60 is derived from the subtraction performed between respective sides of the formulae 58 and 59.

[Formula 60]

$$\begin{pmatrix} x_{R1}'(ts) \\ y_{R1}'(ts) \\ z_{R1}'(ts) \end{pmatrix} - \begin{pmatrix} x_{R2}'(ts) \\ y_{R2}'(ts) \\ z_{R2}'(ts) \end{pmatrix} = \mathbf{Q}'\mathbf{Q}(ts)^{-1} \left\{ \begin{pmatrix} x_{R1}(ts) \\ y_{R1}(ts) \\ z_{R1}(ts) \end{pmatrix} - \begin{pmatrix} x_{R2}(ts) \\ y_{R2}(ts) \\ z_{R2}(ts) \end{pmatrix} \right\}$$

The following formula 61 is derived from multiplication of Q(ts)Q'(-1) by both sides of the formula 60 from the left ("Q'(-1)" indicates the inverse matrix of Q').

[Formula 61]

$$\begin{pmatrix} x_{R1}(ts) \\ y_{R1}(ts) \\ z_{R1}(ts) \end{pmatrix} - \begin{pmatrix} x_{R2}(ts) \\ y_{R2}(ts) \\ z_{R2}(ts) \end{pmatrix} = \mathbf{Q}(ts)\mathbf{Q}'^{-1} \left\{ \begin{pmatrix} x_{R1}'(ts) \\ y_{R1}'(ts) \\ z_{R1}'(ts) \end{pmatrix} - \begin{pmatrix} x_{R2}'(ts) \\ y_{R2}'(ts) \\ z_{R2}'(ts) \end{pmatrix} \right\}$$

Assuming that the respective direction components of the normal vector V(ts) on the radial scan plane on the orthogonal coordinate axes O-xyz are defined as xV(ts), yV(ts) and zV(ts), the following formula 62 is obtained.

[Formula 62]

$$\mathbf{V}(ts) = x_V(ts)\mathbf{i} + y_V(ts)\mathbf{j} + z_V(ts)\mathbf{k}$$

As the V(ts) is the normal vector on the radial scan surface, it becomes orthogonal to the vecor R2R1(ts) with the point R2(ts) as the starting point and the point R1 (ts) as the end point. Accoridngly the following formula 63 is established.

[Formula 63]

$$0 = \mathbf{V}(ts) \cdot \mathbf{R_2 R_1}(ts) = \begin{pmatrix} x_V(ts) & y_V(ts) & z_V(ts) \end{pmatrix} \left\{ \begin{pmatrix} x_{R1}(ts) \\ y_{R1}(ts) \\ z_{R1}(ts) \end{pmatrix} - \begin{pmatrix} x_{R2}(ts) \\ y_{R2}(ts) \\ z_{R2}(ts) \end{pmatrix} \right\}$$

The following formula 64 is obtained by assigning the formula 61 to the {} at the right side of the formula 63.

[Formula 64]

$$\begin{pmatrix} x_V(ts) & y_V(ts) & z_V(ts) \end{pmatrix} \mathbf{Q}(ts) \mathbf{Q}^{'-1} \left\{ \begin{pmatrix} x_{R1}{}'(ts) \\ y_{R1}{}'(ts) \\ z_{R1}{}'(ts) \end{pmatrix} - \begin{pmatrix} x_{R2}{}'(ts) \\ y_{R2}{}'(ts) \\ z_{R2}{}'(ts) \end{pmatrix} \right\} = 0$$

Assuming that the respective direction components of the V'(ts) on the orthogonal coordinate axes O'-x'y'z' are defined as the xV'(ts), yV'(ts) and zV'(ts), the following formula 65 is obtained.

[Formula 65]

$$\mathbf{V}'(ts) = x_V{}'(ts)\mathbf{i}' + y_V{}'(ts)\mathbf{j}' + z_V{}'(ts)\mathbf{k}'$$

The respective direction components are defined as shown in the following formula 66.

[Formula 66]

$$\begin{pmatrix} x_V{}'(ts) & y_V{}'(ts) & z_V{}'(ts) \end{pmatrix} = \begin{pmatrix} x_V(ts) & y_V(ts) & z_V(ts) \end{pmatrix} \mathbf{Q}(ts) \mathbf{Q}^{'-1}$$

The formula 64 may be rewritten to the following formula 67 by the use of the definitional equaltion of the formula 66.

[Formula 67]

$$\begin{pmatrix} x_V{}'(ts) & y_V{}'(ts) & z_V{}'(ts) \end{pmatrix} \left\{ \begin{pmatrix} x_{R1}{}'(ts) \\ y_{R1}{}'(ts) \\ z_{R1}{}'(ts) \end{pmatrix} - \begin{pmatrix} x_{R2}{}'(ts) \\ y_{R2}{}'(ts) \\ z_{R2}{}'(ts) \end{pmatrix} \right\} = 0$$

The formula 67 may further be expressed as shown by the following formula 68.

[Formula 68]

$$\mathbf{V}'(ts) \cdot \mathbf{R}_2' \mathbf{R}_1'(ts) = 0$$

The aforementioned formula 68 indicates that the V'(ts) is always orthogonal to the vector formed by connecting arbitrary two points on the ultrasonic tomographic image marker 71. The V'(ts) given in the formulae 65 and 66 is the normal vector of the ultrasonic tomographic image marker 71. In this way, the normal vector V'(ts) of the ultrasonic tomographic image marker 71 may be obtained through the formulae 65 and 66.

In the explanation referring to Fig. 16, the 3D guide image forming circuit 63 forms the ultrasonic tomographic image marker 71 which is parallelgram provided with the 12 o'clock direction marker 71 a for the tomographic image marker shown in Fig. 18 based on the position and the orientation (center position, normal direction, 12 o'clock direction) of the ultrasonic tomographic image marker 71 obtained in step 557. The 3D guide image forming circuit 63 writes the ultrasonic tomographic image marker 71 into the voxel corresponding to the voxel space in the volume memory 64 based on the position and orientation of the ultrasonic tomographic image marker 71 (step S58). As the extracted data extracted and interpolated by the extraction circuit 62 have been already written in the voxel space, the ultrasonic tomographic image marker 71 and the extracted data are synthesized into the synthetic data (hereinafter referred to as the synthetic data). Fig. 19 is a view showing the synthetic data formed of the ultrasonic tomographic image marker 71 and the extracted data. In Fig. 11, the duodenum is not shown. However, in Fig. 19, the index that indicates the duodenum wall is superimposed on the ultrasonic tomographic image marker 71.

The 3D guide image forming circuit 63 loads the synthetic data from the voxel space within the volume memory 64. The 3D guide image forming circuit 63 deletes the ultrasonic tomographic image marker 71 from the voxel space immediately after the loading.

The 3D guide image forming circuit 63 performs known 3D image processing, for example, shading, addition of shadow, coordinate conversion accompanied with visual line conversion so as to form the 3D guide image data based on the synthetic data. The 3D guide image forming circuit 63 then outputs the 3D guide image data to the mixing circuit 65 (step S60).

The mixing circuit 65 aligns the ultrasonic tomographic image data input from the ultrasonic observation unit 3, and the 3D guide image data input from the 3D guide image forming circuit 63 so as to be output to the display circuit 66 as the mixed data. The display circuit 66 converts the mixed data into the analog video signal so as to be output to the display unit 9. Referring to Fig. 20, the display unit 9 displays the ultrasonic tomographic image 9a2 and the 3D guide image 9al side-by-side (step S61). Fig. 20 is a view showing the display where the ultrasonic tomographic image 9a2 and the 3D guide image 9a1 are laid out side-by-side on the display screen 9a. The respective organs shown on the 3D guide image are displayed, which are coded with colors originally coded by the organs in the reference image data 61a. In the present embodiment shown in Fig. 20, the pancreas, aorta, superior mesenteric vein and duodenal wall are colored by light blue, red, purple and yellow, respectively.

The control circuit 67 confirms whether the operator has commanded to finish the radial scan by pressing the scan control key 12h again while executing the process from steps S55 to S61 (step S62).

If it is confirmed that the operator has not pressed the scan control key 12h, the process returns to step S55 where the aforementioned process is repeatedly executed.

Meanwhile, if it is confirmed that the operator has commanded to finish the radial scan by pressing the scan control key 12h again, the control circuit 67 terminates the process, and outputs the scan control signal for commanding to finish the radial scan control to the ultrasonic observation unit 3. In response to reception of the scan control signal, the ultrasonic observation unit 3 outputs the rotation control signal to the motor 33 so as to stop the rotation. In response to reception of the rotation control signal, the motor 33 stops the rotation of the ultrasonic transducer 31.

Thus, the process from steps S55 to S61 is repeatedly executed as described above to form the new 3D guide image at each cycle including single radial scan performed by the ultrasonic transducer 31, formation of the ultrasonic tomographic image data by the ultrasonic observation unit 3, and input of the ultrasonic tomographic image data to the mixing circuit 65 from the ultrasonic observation unit 3. The 3D guide image on the display screen 9a of the display unit 9 is updated together with the newly formed ultrasonic tomographic image in real time. As the operator manually operates the flexible portion 22 and the rigid portion 21 to move the radial scan plane, the ultrasonic tomographic image marker 71 on the 3D guide image is moved with respect to the extracted data as indicated by the outline arrow 73 shown in Fig. 20.

The operator cannot confirm the position of the components of the ultrasonic diagnostic apparatus, especially the ultrasonic endoscope 1 in the body cavity. As the portion to be inserted into the body cavity of the subject is formed of the flexible material, it is difficult for the operator to accurately confirm the position on the scan plane. Conventionally with the actual use of the ultrasonic diagnostic apparatus, the operator estimates the affected site so as to be displayed as the ultrasonic image. However, reading of the ultrasonic image on the display for analysis requires substantially high skills. Accordingly, this has interfered the wide spread of the ultrasonic endoscope 1.

In embodiment 1, the observation position of the ultrasonic tomographic image corrected in reference to the sample point is superimposed on the 3D guide image formed from the reference image data 61 a using the calculation formula as the ultrasonic tomographic image marker 71 on the assumption that the arrangement of the interest organs on the reference image data 61 a is the same as that of the actual organs of the subject. This makes it possible to easily confirm the observation position on the ultrasonic tomographic image in reference to the 3D guide image.

The operator is allowed to confirm the anatomical position of the subject's body, which corresponds with the site of the ultrasonic tomographic image currently observed while operating the ultrasonic endoscope 1 including the flexible portion 22 in reference to the 3D guide image which is color coded by the respective organs, for example. This makes it possible to perform the accurate diagnosis easily, thus reducing the time for inspection and the time required for the inexperienced operator to learn the operation of the ultrasonic diagnostic apparatus including the ultrasonic endoscope. The medical usability of the ultrasonic diagnostic apparatus of the type where the ultrasonic wave is irradiated from the subject's body as described above is higher than that of the ultrasonic diagnostic apparatus of the type where the ultrasonic wave is irradiated from outside the body. The wide-spread use of the ultrasonic diagnostic apparatus of the type where the ultrasonic wave is irradiated from inside of the subject's body contributes to the development in the medical field.

In the present embodiment, if the triangular pyramid defined by the four sample points has the same positional correlation as that of the triangular pyramid defined by the four characteristic points, the 3D guide image is formed on the assumption that the respective points anatomically correspond. This makes it possible to automatically correct the change in the subject's posture and the difference in the body size, resulting in accurate formation of the 3D guide image.

In the present embodiment, the ultrasonic image and the 3D guide image may be automatically observed together in real time during the radial scan. This allows the operator to easily identify the anatomical correlation between the ultrasonic tomographic image currently observed and the actual site of the body. Even if the scan plane of the ultrasonic endoscope 1 is changed at various angles, the operator is allowed to accurately observe the interest region in reference to the 3D guide image.

In the present embodiment, the 3D guide image is formed by detecting not only the position of the scan plane but also the orientation thereof. If the orientation of the scan plane of the radial scan is changed, the orientation of the ultrasonic tomographic image marker 71 is automatically changed, thus constantly forming accurate 3D guide image accurately. Even if the scan plane of the ultrasonic endoscope 1 is changed at various angles adjacent to the interest region, the operator is allowed to observe the interest region accurately using the 3D guide image.

In addition, in the embodiment, the data having the respective attributes changed, for example, such organs as pancreas, pancreatic duct, choledoch duct, portal preliminarily color-coded are used as the reference image data 61 a such that the image having the color-coded organs are displayed as the 3D guide image. This makes it possible to comprehensively observe the organ serving as the index on the 3D guide image. This further makes it possible to change the scan plane of the ultrasonic endoscope 1 within the body cavity while viewing the 3D guide image. This may reduce the time required for the inspection as the approach to the interest region such as the affected site is accelerated.

Also, in the embodiment, the sample points on the body surface (xiphoid process and right end of pelvis) of the four sample points are detected using the posture detection plate 6 and the marker stick 7. The sample points within the body cavity (pylorus and duodenal papilla) are detected using the receiving coil 42 attached to the tip of the ultrasonic endoscope 1. That is, the sample points on the body surface and the sample points within the body cavity are detected separately. This may reduce the labor for cleaning the ultrasonic endoscope 1 before operation compared with the case where the sample points on the body surface are detected using only the ultrasonic endoscope 1. The sample points within the body cavity may also be detected so as to form the accurate 3D guide image on the assumption that the sample points within the body cavity moves as the interest region is moved therein accompanied with the movement of the endoscope 1. Especially in the case of inspection of pancreas and lung, the sample points adjacent to the interest region may be obtained. The calculation of the position and orientation of the ultrasonic tomographic image marker 71 is assumed to become more accurate as the sample point becomes closer to the interest region, and to the space within the triangular pyramid defined by the sample points compared with the one outside the triangular pyramid. The more accurate 3D guide image may be formed adjacent to the interest region by obtaining the sample points at the appropriate site within the body cavity.

As described above, characteristic points and sample points are set on the xiphoid process, right end of pelvis, pylorus and duodenal papilla adjacent to the head of pancreas intended to be inspected. In the present embodiment, both the characteristic points and the sample points may be set in accordance with the command through the mouse 11 and the keyboard 12 and the output through the position orientation calculation unit 4. Therefore, if the interest region is identified before the operation, it is easier to set the characteristic points and sample points adjacent to the interest region. For example, if the pancreas body is intended to be inspected; the cardia may be added as the characteristic point and the sample point adjacent to the pancreas body. As the interest region becomes adjacent to the sample point and inside the triangular pyramid defined by the sample points, the calculation of position and orientation of the ultrasonic tomogrpahic image marker 71 becomes accurate, resulting in more accurate 3D guide image adjacent to the interest region.

In the embodiment, the posture detection plate 6 is fixed to the subject such that its reference position is overlapped with the xiphoid process in order to form the 3D guide image by obtaining the change in the position and orientation of the posture detection plate 6 constantly and correcting the sample points. This makes it possible to form the accurate 3D guide image irrespective of the change in the posture of the subject while obtaining the sample points and performing the radial scan.

In the generally employed ultrasonic diagnostic apparatus as disclosed in Japanese Unexamined Patent Application Publication No. 2004-113629 as described above, the process for designating the sample points for verifying the position and orientation using the outside image and the ultrasonic image is not clarified. Especially in the ultrasonic diagnostic apparatus of the type for inserting the flexible ultrasonic probe such as the ultrasonic endoscope 1 into the body cavity, the operation of the ultrasonic probe itself may cause the interest organ to move. Accordingly, in the case where the points on the body surface are only used as the sample points to verify the position in reference to the reference image, the resultant guide image becomes inaccurate. In the embodiment, the forceps channel 51 is formed in the ultrasonic endoscope 1 to allow the position detection probe 8 to be inserted through the forceps end 52 of the forceps channel 51 and to protrude through the protruding end 53 such that the tip of the position detection probe 8 is brought into contact with the sample point under the optical visual field to designate the sample point on the body cavity surface. This makes it possible to form the accurate guide image through accurate designation of the sample points on the body cavity surface under the optical visual field.

In the above-described embodiment 1, the ultrasonic diagnostic apparatus is provided with the ultrasonic endoscope 1 including the forceps channel 51 and the position detection probe 8 inserted into the forceps channel 51. However, the configuration is not limited to the one as described above. For example, the ultrasonic endoscope 1 may be configured for the exclusive use where the rigid portion 21 contains the receiving coil 42 therein without the forceps channel 51.

In the above-described embodiment 1, the characteristic points are set in response to the command input through the mouse 11 and the keyboard 12. In the case where the interest region or the protocol of the inspection is predetermined, the set of various types of characteristic points are stored in the reference image memory 61 as the factory default values. In response to the command of the operator input through the mouse 11 and the keyboard 12 via the control circuit 67, the appropriate set of the characteristic points may be loaded form the reference image memory 61 before obtaining the sample points.

Moreover, in the above-described embodiment 1, as the operator presses the predetermined key on the keyboard 12 or clicks on the menu of the screen with the mouse 11 for setting the characteristic points, the reference image data 61a designated with the lowest order from the first, second, third, fourth and the like will be displayed on the screen of the display unit 9. Alternatively, a plurality of reference image data 61a are loaded at one time such that the list of the loaded reference image data 61a is displayed on the display unit 9.

In the above-described embodiment 1, the posture detection plates 6 and the marker sticks 7 are attached to a plurality of predetermined portions of the subject's body, for example, the xiphoid process and the pelvis so as to correct the change in the subject's posture or the difference in the body size, and then the marker stick 7 is removed to leave one of the posture detection plates 6 to correct the change in the subject's posture during the inspection. It is possible to anesthetize the subject just before the operation, and the position of the plurality of points may be measured sequentially using the single marker stick 7 in the immobilized state of the subject. During the inspection, the marker stick 7 is always attached as well as the posture detection plate 6 such that the change in the subject's posture may be corrected. The 3D guide image may further be accurate by attaching the marker stick 7 to the appropriate site of the body of the subject.

In addition, in the above-described embodiment 1, the center position, normal direction and 12 o'clock direction are calculated, based on which the ultrasonic tomographic image marker 71 is obtained. Alternatively, each of four corners of the ultrasonic tomographic image data may be converted using the formula 39 to obtain the four anatomically corresponding points, based on which the ultrasonic tomographic image marker 71 is obtained. The size of the 3D guide image is not derived from the points of four corners of the ultrasonic tomographic image data but is designated by inputting the value of the size through the keyboard 12 or selecting the menu of the size on the screen with the mouse 11, taking the display size and display magnification in account.

Furthermore, in the above-described embodiment 1, the transmission antenna 5 and the receiving coil 42 are used as the position detection means for detecting the position and orientation by the magnetic field. The position and orientation may be detected based on acceleration or other means instead of the magnetic field. The receiving coil 42 may be attached to the position detection probe 8 to be inserted into the body cavity, and the transmission antenna 5 is provided extracorporeally. The configuration may invert positions of the transmission/reception, that is, the transmission antenna is attached to the position detection probe 8, and the receiving coil may be provided extracorporeally.

In the above-described embodiment 1, the origin O is set at the specific position of the transmission antenna 5. However, it may be set to the other position so long as the positional relationship with the transmission antenna 5 is maintained.

Moreover, in the above-described embodiment 1, the ultrasonic endoscope 1 of radial scan type is employed as the ultrasonic endoscope 1. Alternatively, it is possible to employ the ultrasonic endoscope of electronic convex type where a group of the ultrasonic transducers 31 is fan-like provided at one side of the insertion axis as disclosed in Japanese Unexamined Patent Application Publication No. 2004-113629. Accordingly, the present invention is not limited to the scan mode of the ultrasonic wave.

In the above-described embodiment 1, image data classified by the organs at each pixel and color-coded to change the attribute are defined as the reference image data 61 a. However, the attributes may be classified by changing the luminance value instead of the color coding. The data classified with any other mode may be employed.

In the above-described embodiment 1, the respective organs on the 3D image are color-coded to be displayed. The classification may be made with the luminance, brightness, saturation and other modes instead of the color-coding.

(embodiment 2)

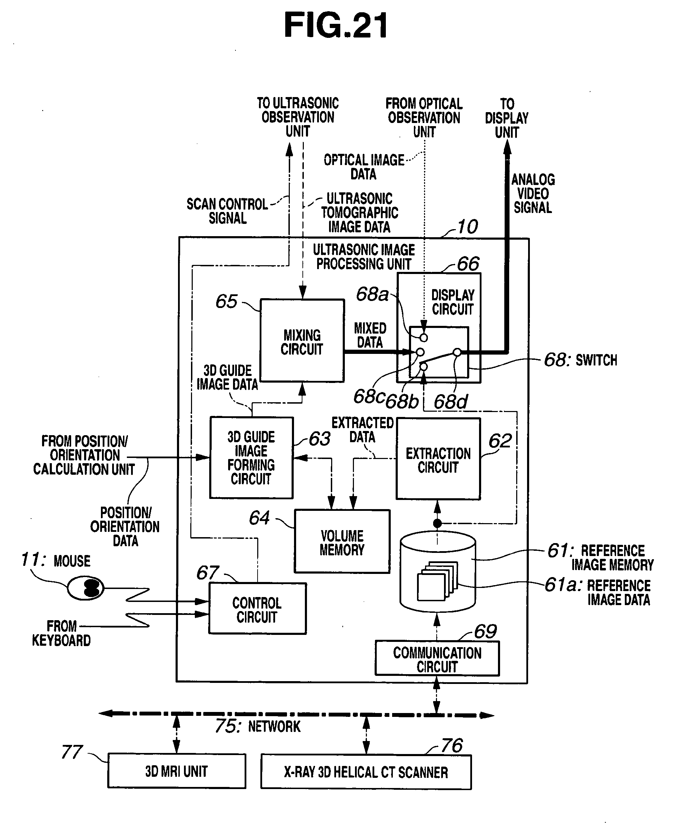

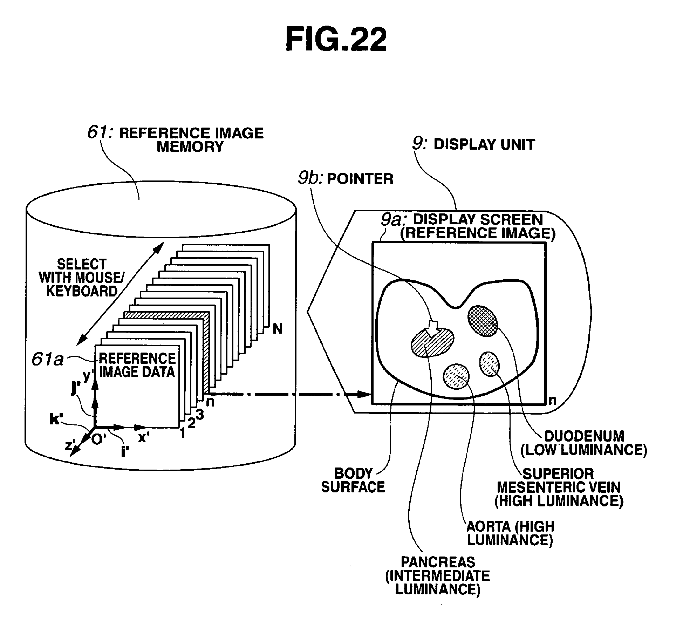

Figs. 21 and 22 show embodiment 2 according to the present invention. Fig. 21 is a block diagram showing the configuration of the ultrasonic image processing unit connected to the external unit.

The same components of embodiment 2 as those of embodiment 1 will be designated with the same reference numerals and explanation thereof, thus, will be omitted. The different feature will only be described hereinafter.

The ultrasonic image processing unit 10 of the present embodiment is formed by adding a communication circuit 69 serving as the communication means to the ultrasonic image processing unit 10 of embodiment 1 as shown in Fig. 1. The communication circuit 69 includes a communication modem that allows the high speed communication of a large volume data.

The communication circuit 69 is connected to the reference image memory 61 and the control circuit 67 for controlling the communication circuit 69.

The communication circuit 69 is further connected to a high-speed network 75, for example, optical communication, ADSL and the like. The network 75 is connected to an X-ray 3D helical CT scanner 76 and a 3D MRI unit 77 as the external unit of the ultrasonic diagnostic apparatus. The communication circuit 69 allows the image data received from the external units to be stored in the reference image memory 61 as the reference image data 61a.

Other configurations are the same as those of embodiment 1.

Next, the operation in embodiment 2, which is different from embodiment 1 will be described. Generally, the present embodiment is different from embodiment 1 in the operation for obtaining the reference image data 61 a and the operation for extracting the interest organ.

The operator preliminarily obtains the reference image data 61 a that cover the entire abdominal area of the subject using the X-ray 3D helical CT (Computer Tomography) scanner and the 3D MRI (Magnetic Resonance Imaging) unit.

Thereafter, upon inspection of the subject using the ultrasonic diagnostic apparatus, the operator presses the predetermined key on the keyboard 12 or clicks the menu on the screen of the display unit 9 with the mouse 11 to command to obtain the reference image data 61 a. Simultaneously, the operator commands to determine the external unit that supplies the data. In response to the command, the control circuit 67 commands the communication circuit 69 to load the reference image data 61 a and the external unit that supplies the data.

If the X-ray 3D helical CT scanner 76 is selected as the external unit that supplies the data, for example, the communication circuit 69 loads a plurality of 2D CT images from the network 75 so as to be stored in the reference image memory 61 as the reference image data 61a. When the image is picked up by the X-ray 3D helical CT scanner 76, the radio-contrast agent is preliminarily infused through the vein of the subject such that the blood vessel, for example, aorta, superior mesenteric vein, and the organ including a large number of blood vessels are displayed at higher luminance on the 2D CT image so as to be discriminated from the peripheral tissue with respect to the luminance.

Meanwhile, in the case where the 3D MRI unit 77 is selected, for example, the communication circuit 69 loads a plurality of 2D MRI images from the network 75 so as to be stored in the reference image memory 61 as the reference image data 61a. When the image is picked up by the 3D MRI unit 77, the radio-contrast agent for MRI which exhibits the high sensitivity with respect to magnetic nuclear resonance is preliminarily infused through the vein of the subject such that the blood vessel, for example, aorta, superior mesenteric vein, and the organ including a large number of blood vessels are displayed at higher luminance on the 2D MRI image so as to be discriminated from the peripheral tissue with respect to the luminance.

As the operation resulting from selecting the X-ray 3D helical CT scanner 76 is the same as the one resulting from selecting the 3D MRI unit 77, the following operation will be described only in the case where the X-ray 3D helical CT scanner 76 is selected as the unit for supplying the data, and the communication circuit 69 loads the plurality of the 2D CT images as the reference image data 61 a.

Fig. 22 is a view showing designation of the interest organ on the reference image loaded from the reference image memory 61 to be displayed.

Fig. 22 is a view of the nth image of the reference image data 61a displayed on the display screen 9a likewise embodiment 1. The radio-contrast agent functions in displaying the blood vessel such as the aorta and superior mesenteric vein with the luminance at the high level, the organ that includes a large number of peripheral blood vessels such as pancreas with the luminance at the intermediate level, and the duodenum with the luminance at the low level, respectively.

The extraction circuit 62 loads all the reference image data 61 a from the first to the Nth images from the reference image memory 61.

The extraction circuit 62 allocates the red color to the blood vessel (aorta, superior mesenteric vein) with the luminance at the high level, the light blue color to the pancreas with the luminance at the intermediate level, and the yellow color to the duodenum with the luminance at the low level with respect to all the first to the Nth images of the reference image data 61 a in accordance with the luminance value such that the reference image is independently extracted.

The extraction circuit 62 allows the color coded images to be stored in the reference image memory 61 as the reference image data 61 a again.

Other operations are the same as those of embodiment 1.

In embodiment 2, the ultrasonic diagnostic apparatus is connected to such external unit as the X-ray 3D helical CT scanner 76 and the 3D MRI unit 77 to input the plurality of the 2D CT images or the plurality of the 2D MRT images so as to be used as the reference image data 61 a. The 3D guide images are formed from the data of the subject. Accordingly, the 3D guide images are expected to be further accurate. This makes it possible to select the data showing interest region most clearly as the reference image data 61a, and to display the 3D guide image that can be easily observed.

Moreover, in the present embodiment, the radio-contrast agent is preliminarily used to pick up the reference image data 61 a in which the blood vessel or the organ that contains a large number of blood vessels has luminance at the higher level than that of the peripheral tissue. The extraction circuit 62 allocates different colors to the images of the reference image data 61 a depending on the luminance value, specifically by different attributes, that is, the blood vessel (aorta, superior mesenteric vein) with the luminance at the high level, the pancreas with the luminance at the intermediate level, and the duodenum with the luminance at the low level so as to be independently extracted. This makes it possible to easily extract the data of such organ as the blood vessel and pancreas. As a result, the 3D guide image having the boundary between the organs easily identified may be formed.

The present embodiment provides the same effects as those obtained in embodiment 1.

In the above-described embodiment 2, a plurality of the 2D CT images are used as the reference image data 61 a. Alternatively, a large number of sliced 2D CT images are superimposed to reconfigure the dense volume data so as to be used as the reference image data 61a.

In the above-described embodiment 2, a plurality of 2D CT images and the 2D MRI images are used as the reference image data 61a. The 3D image data preliminarily obtained from data of the subject using the ultrasonic endoscope 1 which contains the ultrasonic transducer 31 as described above may be employed as the reference image data 61a. Further, the 3D image data preliminarily obtained using other modality, for example, PET (Position Emission Tomography) may be employed. Additionally, the 3D image data preliminarily obtained using the extracorporeal ultrasonic diagnostic apparatus of the type for irradiating the ultrasonic wave extracorporeally may be employed.

Note that, the modified example described in embodiment 1 may be employed in embodiment 2.

(embodiment 3)

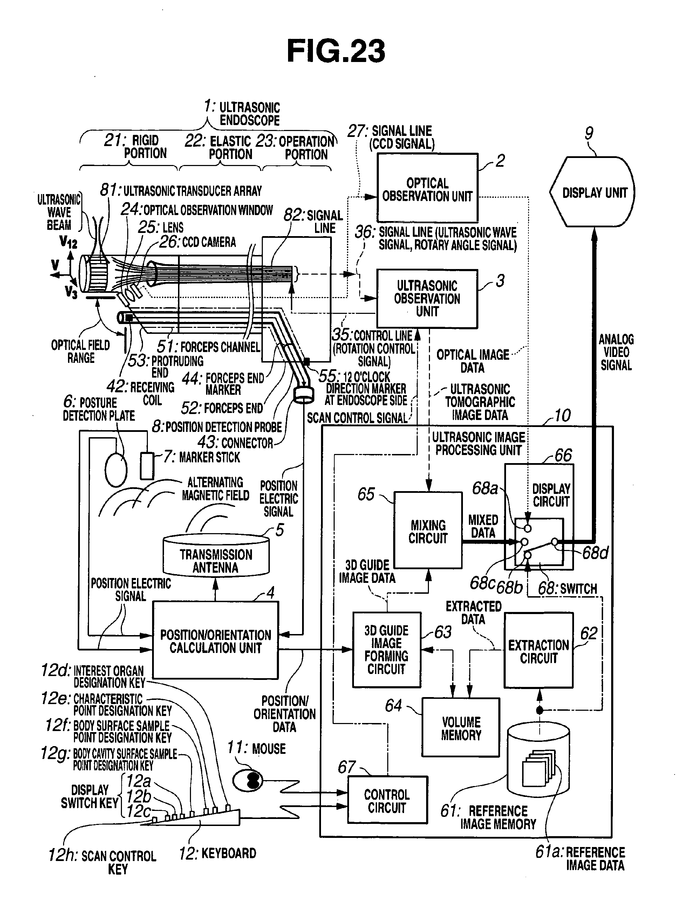

Fig. 23 is a view of embodiment 3 according to the present invention showing a block diagram of the configuration of the ultrasonic diagnostic apparatus.

In embodiment 3, the same components as those shown in embodiments 1 and 2 will be designated with the same reference numerals, and the explanations thereof will be omitted. Only the different features will be described hereinafter.

The ultrasonic diagnostic apparatus of embodiment 3 is different from the one described in embodiment 1 shown in Fig. 1 in the points as described below.

The ultrasonic diagnostic apparatus of embodiment 1 employs the ultrasonic endoscope of mechanical radial scan type, which is provided with the flexible shaft 32 for the flexible portion 22, and the motor 33 and the rotary encoder 34 for the operation portion 23. The ultrasonic diagnostic apparatus of embodiment 3 employs the ultrasonic endoscope 1 of electronic radial scan type which is not provided with the flexible shaft 32, the motor 33 and the rotary encoder 34.

Referring to Fig. 23, the rigid portion 21 in the present embodiment is provided with an ultrasonic transducer array 81 instead of the ultrasonic transducer 31 shown in Fig. 1. The ultrasonic transducer array 81 is configured by arranging the group of tiny ultrasonic transducers each cut into a strip shape along the insertion axis annularly around the insertion axis. The respective ultrasonic transducers that form the ultrasonic transducer array 81 are connected to the ultrasonic observation unit 3 through the signal line 82 via the operation portion 23.

The other configurations are the same as those of embodiment 1 as described above.

The operation in embodiment 3, which is different from that of embodiment 1 will be described. Generally, the present embodiment is different from embodiment 1 in the operation for obtaining the ultrasonic tomographic image, especially for the radial scan.

The ultrasonic observation unit 3 transmits the pulse voltage excitation signal only to the plurality of the ultrasonic transducers as a part of those forming the ultrasonic transducer array 81. In response to reception of the excitation signal, the ultrasonic transducers convert the signal into the ultrasonic wave as the compressional wave of the medium.

The ultrasonic observation unit 3 delays the respective excitation signals such that those signals reach the corresponding ultrasonic transducers at different times. More specifically, the delay is made to form the beam of the single ultrasonic wave when the ultrasonic waves excited by the respective ultrasonic transducers are superimposed within the body of the subject. The ultrasonic wave formed as the beam is irradiated into the body of the subject. The reflected wave from the body resulting from the irradiation passes on the inverse path on which the irradiation is made to reach the respective ultrasonic transducers. The respective ultrasonic transducers convert the reflected wave into the electric echo signal so as to be transmitted to the ultrasonic observation unit 3 on the inverse path of the excitation signal.

Then, the ultrasonic observation unit 3 selects a plurality of ultrasonic transducers relevant to formation of the ultrasonic wave beam so as to radially scan in the plane (radial scan plane) perpendicular to the insertion axis of the rigid portion 21 and the flexible portion 22. The excitation signal is further transmitted to the selected ultrasonic transducers again. This may change the angle in the direction where the ultrasonic beam is irradiated. The aforementioned process is repeatedly executed to perform so-called electronic radial scan.

In embodiment 1, the rotation angle signal from the rotary encoder 34 determines the direction to which the ultrasonic tomographic image data in 12 o'clock direction is orientated with respect to the ultrasonic endoscope 1 for forming the ultrasonic tomographic image data by the ultrasonic observation unit 3. In the present embodiment, the ultrasonic observation unit 3 re-selects the plurality of ultrasonic transducers relevant to the formation of the ultrasonic wave beam to transmit the excitation signal again. The 12 o'clock direction of the ultrasonic tomographic image data may be determined depending on the ultrasonic transducers selected by the ultrasonic observation unit 3 with respect to the 12 o'clock direction.

The other operations are the same as those of embodiment 1 as described above.

In embodiment 1, the mechanical radial scan for rotating the ultrasonic transducer 31 is employed, which may cause the flexible shaft 32 to be twisted. Owing to the twisting in the flexible shaft 32, the angle output from the rotary encoder 34 may deviate from that of the actual ultrasonic transducer 31. This may further cause the deviation in the 12 o'clock direction between the ultrasonic tomographic image and the 3D guide image.

In embodiment 3, the ultrasonic endoscope 1 for performing the electronic radial scan is employed such that the 12 o'clock direction of the ultrasonic tomographic image may be determined depending on the ultrasonic transducer selected by the ultrasonic observation unit 3 with respect to the 12 o'clock direction. This may prevent the deviation in the 12 o'clock deviation. This makes it possible to reduce the deviation in the 12 o'clock deviation that may occur between the ultrasonic tomographic image marker 71 on the 3D guide image displayed on the display unit 9 or the 12 o'clock direction marker 71 a for the tomographic image marker, and the ultrasonic tomographic image, thus structuring the accurate 3D guide image.

The other effects are the same as those obtained in embodiment 1.

Note that, in embodiment 3, the ultrasonic transducer array 81 is attached to the tip of the rigid portion 21 of the ultrasonic endoscope 1. The ultrasonic transducer array 81 may be provided to cover the entire periphery at 360°, but it may be provided at the smaller angles, for example, 270° and 180° for covering a part of the circumferential direction of the rigid portion 21.

Moreover, the modified example described in embodiment 1 may be employed in embodiment 3.

It is to be understood that the present invention is not limited to the aforementioned embodiments, but may be modified or applied so as not to deviate from the scope of the present invention.

**Claims**

**1.** An ultrasonic diagnostic apparatus comprising:

ultrasonic tomographic image forming means that forms an ultrasonic tomographic image based on an ultrasonic signal obtained by transmission and reception of an ultrasonic wave to and from inside of a living body;
detection means that detects a position and/or an orientation of the ultrasonic tomographic image;
reference image data storage means that stores reference image data;
three-dimensional guide image forming means that forms a stereoscopic three-dimensional guide image for guiding an anatomical position and/or orientation of the ultrasonic tomographic image using the position and/or orientation detected by the detection means based on the reference image data stored in the reference image data storage means; and
display means that displays the three-dimensional guide image formed by the three-dimensional guide image forming means.

2. The ultrasonic diagnostic apparatus according to Claim 1, further comprising extraction means that extracts a specific region from the reference image data stored in the reference image data storage means, wherein the three-dimensional guide image forming means forms the three-dimensional guide image by superimposing an ultrasonic tomographic image marker that indicates a position and an orientation of the ultrasonic tomographic image on the stereoscopic image based on the region extracted by the extraction means.

3. The ultrasonic diagnostic apparatus according to Claim 1, further comprising sample point position detection means that detects a position of a sample point of the living body, wherein the three-dimensional guide image forming means forms the three-dimensional guide image by performing a verification between a position of the sample point detected by the sample point position detection means and a position of a characteristic point on the reference image data stored in the reference image data storage means.

4. The ultrasonic diagnostic apparatus according to Claim 3, wherein the display means displays at least a portion of the reference image data stored in the reference image data storage means, and further includes characteristic point designation means that designates a position of the characteristic point on the reference image data displayed by the display means.

5. The ultrasonic diagnostic apparatus according to Claim 3, wherein:

the sample point position detection means includes body cavity sample point position detection means that detects a position of the sample point within a body cavity of the living body; and
the body cavity sample point position detection means is disposed at a tip portion of an ultrasonic probe inserted into the body cavity.

6. The ultrasonic diagnostic apparatus according to Claim 5, wherein the detection means serves as the body cavity sample point position detection means.

7. The ultrasonic diagnostic apparatus according to Claim 6, wherein the sample point position detection means is provided separately from the body cavity sample point position detection means, and further includes body surface sample point position detection means that detects a position of the sample point on a surface of the living body.

8. The ultrasonic diagnostic apparatus according to Claim 3, wherein the sample points are set for four points selected from a xiphoid process, a right end of pelvis, a pylorus, a duodenal papilla and a cardia.

9. The ultrasonic diagnostic apparatus according to Claim 3, further comprising:

posture detection means that detects a position or a posture of the living body; and
sample point position correction means that corrects the position of the sample point detected by the sample point position detection means using the position or the posture detected by the posture detection means, wherein the three-dimensional guide image forming means performs a verification between a position of the sample point corrected by the sample point position correction means and a position of the characteristic point on the reference image data stored in the reference image data storage means to form the three-dimensional guide image.

10. The ultrasonic diagnostic apparatus according to Claim 9, wherein the sample point position detection means includes body cavity sample point position detection means that detects a position of the sample point in the body cavity of the living body, and body surface sample point position detection means that is provided separately from the body cavity sample point position detection means to detect a position of the sample point on a body surface of the living body, wherein the posture detection means serves as the body surface sample point position detection means.

11. The ultrasonic diagnostic apparatus according to Claim 2, wherein:

the reference image data stored in the reference image data storage means are obtained through an image pickup operation performed by an external image pickup device using a radio-contrast agent; and
the extraction means extracts a specific region from the reference image data stored in the reference image data storage means based on a luminance value of the reference image data obtained by a use of the radio-contrast agent.

12. The ultrasonic diagnostic apparatus according to Claim 2, wherein:

the display means displays at least a portion of the reference image data stored in the reference image data storage means;
interest region designation means that designates a portion of the specific region on the reference image data displayed by the display means is provided; and
the extraction means extracts the specific region designated by the interest region designation means.

13. The ultrasonic diagnostic apparatus according to Claim 1, wherein the ultrasonic tomographic image forming means forms an ultrasonic tomographic image based on an ultrasonic signal output from an ultrasonic probe including an insertion portion having a flexibility to be inserted into the body cavity of the living body, and an ultrasonic transducer that is disposed at a tip portion of the insertion portion to transmit and receive the ultrasonic wave to and from the living body.

14. The ultrasonic diagnostic apparatus according to Claim 13, wherein the ultrasonic transducer performs a scan operation in a plane orthogonal to an insertion axis of the ultrasonic probe.

15. The ultrasonic diagnostic apparatus according to Claim 13, wherein the ultrasonic transducer is formed as an ultrasonic transducer array that electronically performs a scan operation.

16. The ultrasonic diagnostic apparatus according to Claim 1, wherein the reference image data stored in the reference image data storage means are image data which are classified by respective regions.

17. The ultrasonic diagnostic apparatus according to Claim 1, further comprising communication means that obtains image data picked up by an external image pickup device as the reference image data, wherein the reference image data storage means stores reference image data obtained by the communication means.

18. The ultrasonic diagnostic apparatus according to Claim 17, wherein the communication means is connected to at least one kind of the external image pickup devices via a network through which the reference image data are obtained.

19. The ultrasonic diagnostic apparatus according to Claim 17, wherein the external image pickup device is formed as at least one of an X-ray CT scanner, an MRI unit, a PET unit, and an ultrasonic diagnostic unit.

20. The ultrasonic diagnostic apparatus according to any one of Claims 1 to 19, wherein the display means displays the ultrasonic tomographic image formed by the ultrasonic tomographic image forming means and the three-dimensional guide image formed by the three-dimensional guide image forming means simultaneously.

21. The ultrasonic diagnostic apparatus according to any one of Claims 1 to 20, wherein the three-dimensional guide image forming means forms the three-dimensional guide image on a real time basis together with formation of the ultrasonic tomographic image performed by the ultrasonic tomographic image forming means based on an ultrasonic signal obtained by transmission and reception of the ultrasonic wave to and from inside of the living body.

22. An ultrasonic diagnostic apparatus comprising:

ultrasonic tomographic image forming means that forms an ultrasonic tomographic image based on an ultrasonic signal obtained by transmission and reception of an ultrasonic wave to and from inside of a living body;
detection means that detects a position and/or an orientation of the ultrasonic tomographic image;

reference image data storage means that stores reference image data;

a position detection probe including sample point position detection means that detects a position of a sample point of the living body;

an ultrasonic endoscope provided with a channel which allows the position detection probe to be inserted therethrough and an optical observation window for obtaining the ultrasonic signal;

guide image forming means that forms a guide image to guide an anatomical position and/or orientation of the ultrasonic tomographic image by performing a verification between a position of the sample point detected by the sample point position detection means in a state where the position detection probe protrudes from the channel to be in an optical field range of the optical observation window and a position of a characteristic point on the reference image data stored in the reference image data storage means using a position and/or an orientation detected by the detection means; and

display means that displays the guide image formed by the guide image forming means.

# FIG.1

# FIG.2

42: RECEIVING COIL
8: POSITION DETECTION PROBE
41: OUTER BARREL
45: 12 O'CLOCK DIRECTION MARKER AT PROBE SIDE
43: CONNECTOR
46: SIGNAL LINE
TO POSITION/ ORIENTATION CALCULATION UNIT
44: FORCEPS END MARKER

# FIG.3

6: POSTURE DETECTION PLATE
6a: PLATE COIL
REFERENCE POSITION L
6b: PLATE COIL
6c: PLATE COIL
6d
TO POSITION/ORIENTATION CALCULATION UNIT

41

# FIG.4

7: MARKER STICK

REFERENCE POSITION M

7a: MARKER COIL

TO POSITION/ORIENTATION CALCULATION UNIT

# FIG.5

61: REFERENCE IMAGE MEMORY

N

DUODENUM (YELLOW)

PANCREAS (LIGHT BLUE)

61a: REFERENCE IMAGE DATA

y'

j'

k'

3

2

1

O'    x'

z'    i'

SUPERIOR MESENTERIC VEIN (PURPLE)

AORTA (RED)

# FIG.6

VOXEL SPACE

y'

x'

z'

O'

VOXEL

# FIG.7

z

i    k    j

x    O    y

5: TRANSMISSION
ANTENNA

# FIG.8

```
                    ┌───────────┐
                    │   START   │
                    └─────┬─────┘
                          ▼                               ╱S1
        ┌─────────────────────────────────────────────────┐
        │     INTEREST ORGAN EXTRACTION PROCESS            │
        └─────────────────────┬───────────────────────────┘
                              ▼                           ╱S2
        ┌─────────────────────────────────────────────────┐
        │   CHARACTERISTIC POINT DESIGNATION PROCESS       │
        └─────────────────────┬───────────────────────────┘
                              ▼                           ╱S3
        ┌─────────────────────────────────────────────────┐
        │      SAMPLE POINT DESIGNATION PROCESS            │
        └─────────────────────┬───────────────────────────┘
                              ▼                           ╱S4
        ┌─────────────────────────────────────────────────┐
        │    3D GUIDE IMAGE FORMING/DISPLAY PROCESS        │
        └─────────────────────┬───────────────────────────┘
                              ▼
                    ┌───────────┐
                    │    END    │
                    └───────────┘
```

# FIG.9

```
┌─────────────────────────────────────────────┐
│     INTEREST ORGAN EXTRACTION PROCESS         │
└─────────────────────────────────────────────┘
                      │
  S11                 ▼
┌─────────────────────────────────────────────┐
│        PRESS DISPLAY SWITCH KEY 12b           │
└─────────────────────────────────────────────┘
                      │
                      ▼         S12
┌─────────────────────────────────────────────┐
│           LOAD REFERENCE IMAGE                │
└─────────────────────────────────────────────┘
                      │          S13
                      ▼
┌─────────────────────────────────────────────┐
│         DISPLAY REFERENCE IMAGE               │
└─────────────────────────────────────────────┘
                      │
                      ▼              S14
          ◇─────────────────────────◇
  YES      INTEREST ORGAN SHOWN?
          ◇─────────────────────────◇
                      │ NO
  S15                 ▼
┌─────────────────────────────────────────────┐
│         SELECT REFERENCE IMAGE                │
└─────────────────────────────────────────────┘

  S16                 ▼
┌─────────────────────────────────────────────┐
│         DESIGNATE INTEREST ORGAN              │
└─────────────────────────────────────────────┘
                      │          S17
                      ▼
┌─────────────────────────────────────────────┐
│          EXTRACT INTEREST ORGAN               │
└─────────────────────────────────────────────┘
                      │          S18
                      ▼
┌─────────────────────────────────────────────┐
│      INTERPOLATE EXTRACTED PIXEL DATA         │
└─────────────────────────────────────────────┘
                      │          S19
                      ▼
┌─────────────────────────────────────────────┐
│          WRITE EXTRACTED DATA                 │
└─────────────────────────────────────────────┘
                      │
                      ▼
              ┌──────────────┐
              │    RETURN     │
              └──────────────┘
```

# FIG.10

**61:** REFERENCE IMAGE
MEMORY

**9:** DISPLAY UNIT

**9b:** POINTER

**9a:** DISPLAY SCREEN
(REFERENCE IMAGE)

SELECT
WITH MOUSE/
KEYBOARD

**61a** — REFERENCE
IMAGE DATA

N

DUODENUM
(YELLOW)

BODY
SURFACE

SUPERIOR
MESENTERIC
VEIN (PURPLE)

AORTA (RED)

PANCREAS
(LIGHT BLUE)

# FIG.11

AORTA (RED)

SUPERIOR
MESENTERIC
VEIN (PURPLE)

PANCREAS
(LIGHT BLUE)

# FIG.12

CHARACTERISTIC POINT DESIGNATION PROCESS

S21 — PRESS DISPLAY SWITCH KEY 12b

S22 — LOAD REFERENCE IMAGE

S23 — DISPLAY REFERENCE IMAGE

S24 — CHARACTERISTIC POINT SHOWN?

YES

NO

S25 — SELECT REFERENCE IMAGE

S26 — DESIGNATE CHARACTERISTIC POINT

S27 — WRITE DIRECTION COMPONENT OF POSITION VECTOR OF CHARACTERISTIC POINT

S28 — DESIGNATION OF 4 CHARACTERISTIC POINTS FINISHED?

NO

YES

RETURN

EP 1 815 797 A1

# FIG.13

61: REFERENCE IMAGE MEMORY

9: DISPLAY UNIT

9a: DISPLAY SCREEN (REFERENCE IMAGE)

9b: POINTER

BODY SURFACE

POINT $P_0'$ (XIPHOID PROCESS)

SELECT WITH MOUSE/ KEYBOARD

61a

REFERENCE IMAGE DATA

# FIG.14

```
( SAMPLE POINT DESIGNATION PROCESS )
                    │
S31 ─┐              ▼
┌──────────────────────────────────────────────┐
│        PRESS DISPLAY SWITCH KEY 12a           │
└──────────────────────────────────────────────┘
                    │                  S32
                    ▼
<          DISPLAY OPTICAL IMAGE                 >
                    │                  S33
                    ▼
┌──────────────────────────────────────────────┐
│ CONTACT REFERENCE POSITION OF MARKER STICK/   │
│ POSTURE DETECTION PLATE WITH SAMPLE POINT ON  │
│               BODY SURFACE                     │
└──────────────────────────────────────────────┘
S34 ─┐              ▼
┌──────────────────────────────────────────────┐
│ PRESS BODY SURFACE SAMPLE POINT DESIGNATION KEY│
└──────────────────────────────────────────────┘
                    │                  S35
                    ▼
/         LOAD POSITION/ORIENTATION DATA        /
                    │                  S36
                    ▼
/ WRITE DIRECTION COMPONENT OF POSITION VECTOR OF /
/ SAMPLE POINT ON BODY SURFACE AND ROTATING MATRIX/
                    │                  S37
                    ▼
┌──────────────────────────────────────────────┐
│ MOVE RIGID PORTION TO SEARCH SAMPLE POINT ON  │
│             BODY CAVITY SURFACE                │
└──────────────────────────────────────────────┘
                    │                  S38
                    ▼
┌──────────────────────────────────────────────┐
│PROTRUDE POSITION DETECTION PROBE FROM PROTRUDING│
│ END TO BE IN CONTACT WITH SAMPLE POINT ON BODY │
│             CAVITY SURFACE                     │
└──────────────────────────────────────────────┘
S39 ─┐              ▼
┌──────────────────────────────────────────────┐
│ PRESS BODY CAVITY SURFACE SAMPLE POINT        │
│             DESIGNATION KEY                    │
└──────────────────────────────────────────────┘
                    │                  S40
                    ▼
/         LOAD POSITION/ORIENTATION DATA        /
                    │                  S41
                    ▼
/ WRITE DIRECTION COMPONENT OF POSITION VECTOR OF /
/ SAMPLE POINT ON BODY CAVITY SURFACE AND ROTATING/
/               MATRIX                           /
                    │
                    ▼
              ( RETURN )
```

# FIG.15

9a: **DISPLAY SCREEN**
**(OPTICAL IMAGE)**

**DUODENAL**
**PAPILLA**

8: **POSITION**
**DETECTION**
**PROBE**

# FIG.16

$\big($ 3D GUIDE IMAGE FORMING/DISPLAY PROCESS $\big)$

**S51**
FIX POSITION DETECTION PROBE TO PREDETERMINED POSITION

**S52**
PRESS DISPLAY SWITCH KEY 12c

**S53**
LOAD EACH DIRECTION COMPONENT OF POSITION VECTORS AND ROTATING MATRIX OF 4 CHARACTERISTIC POINTS AND 4 SAMPLE POINTS

**S54**
START RADIAL SCAN

**S55**
LOAD POSITION/ORIENTATION DATA

**S56**
CORRECT SAMPLE POINT POSITION IN RESPONSE TO CHANGE IN POSTURE OF SUBJECT

**S57**
CALCULATE POSITION/ORIENTATION OF RADIAL SCAN PLANE

**S58**
WRITE ULTRASONIC TOMOGRAPHIC IMAGE MARKER

**S59**
LOAD SYNTHETIC DATA AND DELETE ULTRASONIC TOMOGRAPHIC IMAGE MARKER

**S60**
FORM 3D GUIDE IMAGE DATA

**S61**
DISPLAY ULTRASONIC TOMOGRAPHIC IMAGE AND 3D GUIDE IMAGE

**S62**
RADIAL SCAN FINISHED? — NO

YES

$\big($ RETURN $\big)$

# FIG.17

(A)

(B)

# FIG.18

71a: 12 O'CLOCK DIRECTION MARKER FOR TOMOGRAPHIC IMAGE MARKER

71: ULTRASONIC TOMOGRAPHIC IMAGE MARKER

WRITE INTO VOXEL SPACE

# FIG.19

AORTA (RED)

SUPERIOR MESENTERIC VEIN (PURPLE)

PANCREAS (LIGHT BLUE)

DUODENAL WALL

71: ULTRASONIC TOMOGRAPHIC IMAGE MARKER

71a: 12 O'CLOCK DIRECTION MARKER FOR TOMOGRAPHIC IMAGE MARKER

# FIG.20

9a: DISPLAY SCREEN (3D GUIDE IMAGE
AND ULTRASONIC TOMOGRAPHIC
IMAGE)

9a1: 3D GUIDE
IMAGE

9a2: ULTRASONIC
TOMOGRAPHIC
IMAGE

73

PANCREAS
(LIGHT BLUE)

DUODENAL
WALL

SUPERIOR
MESENTERIC
VEIN (PURPLE)

AORTA

71: ULTRASONIC
TOMOGRAPHIC
IMAGE MARKER

DUODENAL
WALL
(YELLOW)

AORTA
(RED)

PANCREAS

71a: 12 O'CLOCK DIRECTION
MARKER FOR TOMOGRAPHIC
IMAGE MARKER

# FIG.21

EP 1 815 797 A1

# FIG.22

61: REFERENCE IMAGE MEMORY

9: DISPLAY UNIT

9b: POINTER

9a: DISPLAY SCREEN (REFERENCE IMAGE)

SELECT WITH MOUSE/ KEYBOARD

61a REFERENCE IMAGE DATA

N

DUODENUM (LOW LUMINANCE)

SUPERIOR MESENTERIC VEIN (HIGH LUMINANCE)

AORTA (HIGH LUMINANCE)

BODY SURFACE

PANCREAS (INTERMEDIATE LUMINANCE)

# FIG.23

## INTERNATIONAL SEARCH REPORT

| | International application No. |
|---|---|
| | PCT/JP2005/021576 |

| A. CLASSIFICATION OF SUBJECT MATTER |
|---|
| ***A61B8/12*** (2006.01) |

According to International Patent Classification (IPC) or to both national classification and IPC

| B. FIELDS SEARCHED |
|---|

Minimum documentation searched (classification system followed by classification symbols)
A61B8/00-8/15

Documentation searched other than minimum documentation to the extent that such documents are included in the fields searched

| Jitsuyo Shinan Koho | 1922-1996 | Jitsuyo Shinan Toroku Koho | 1996-2006 |
|---|---|---|---|
| Kokai Jitsuyo Shinan Koho | 1971-2006 | Toroku Jitsuyo Shinan Koho | 1994-2006 |

Electronic data base consulted during the international search (name of data base and, where practicable, search terms used)
ICHUSHI WEB

| C. DOCUMENTS CONSIDERED TO BE RELEVANT |
|---|

| Category* | Citation of document, with indication, where appropriate, of the relevant passages | Relevant to claim No. |
|---|---|---|
| X | JP 2004-230194 A (Olympus Corp.), 19 August, 2004 (19.08.04), Particularly, Fig. 12 (Family: none) | 1,2,11, 13-15,20 |
| X __ A | JP 2003-038492 A (Pentax Kabushiki Kaisha, The Jikei University), 12 February, 2003 (12.02.03), Particularly, Fig. 1 & US 2002/0183592 A1 | 1,2,13,15, 20 11,14 |
| A | OOKUMA et al., "Real-time virtual sonography ni Tsuite", Journal of Medical Ultrasonics, 15 April, 2003 (15.04.03), Vol.30, special extra issue, page S277 | 1,2,11, 13-15,20 |

☐ Further documents are listed in the continuation of Box C.  ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 02 February, 2006 (02.02.06) | 14 February, 2006 (14.02.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

## INTERNATIONAL SEARCH REPORT

| International application No. |
| --- |
| PCT/JP2005/021576 |

**Box No. II       Observations where certain claims were found unsearchable (Continuation of item 2 of first sheet)**

This international search report has not been established in respect of certain claims under Article 17(2)(a) for the following reasons:

1. ☐ Claims Nos.:
    because they relate to subject matter not required to be searched by this Authority, namely:

2. ☐ Claims Nos.:
    because they relate to parts of the international application that do not comply with the prescribed requirements to such an extent that no meaningful international search can be carried out, specifically:

3. ☐ Claims Nos.:
    because they are dependent claims and are not drafted in accordance with the second and third sentences of Rule 6.4(a).

**Box No. III       Observations where unity of invention is lacking (Continuation of item 3 of first sheet)**

This International Searching Authority found multiple inventions in this international application, as follows:

    See extra sheet.

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☒ No required additional search fees were timely paid by the applicant.   Consequently, this international search report is restricted to the invention first mentioned in the claims; it is covered by claims Nos.: 1, 2, 11, 13-15, 20

**Remark on Protest**
the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

| **INTERNATIONAL SEARCH REPORT** | International application No. |
|---|---|
| | PCT/JP2005/021576 |

Continuation of Box No.III of continuation of first sheet(2)

The technical feature common to the inventions of claims 1-22 is the feature described in claim 1. However, the search has revealed that the technical feature is not novel since it is disclosed in document JP 2004-230194 A (Olympus Optical Co., Ltd.), 19 August, 2004 (19.08.04).

Accordingly, the aforementioned technical feature cannot be a special technical feature within the meaning of PCT Rule 13.2, second sentence.

Consequently, it is obvious that the inventions of claims 1, 2, 11, 13-15, 20, the inventions of claims 3-10, the invention of claim 12, the invention of claim 16, the inventions of claims 17-19, and the invention of claim 22 do not satisfy the requirement of unity of invention.

Form PCT/ISA/210 (extra sheet) (April 2005)

**REFERENCES CITED IN THE DESCRIPTION**

**Patent documents cited in the description**

- JP 10151131 A

- JP 2004113629 A